# FASCICULE DE BREVET EUROPEEN

(11) **EP 3 154 466 B1**
(45) Date de publication et mention de la délivrance du brevet: **23.11.2022**
(21) Numéro de dépôt: 15733825.2
(22) Date de dépôt: 12.06.2015
(51) Int. Cl.: A61B 90/00, A61M 25/01

(54) **MODULE ROBOTISE D'ENTRAÎNEMENT D'ORGANE MÉDICAL SOUPLE ALLONGE**
ROBOTISIERTES MODUL ZUR FÜHRUNG EINER LANGGESTRECKTEN FLEXIBLEN MEDIZINISCHEN VORRICHTUNG
ROBOTIZED MODULE FOR GUIDING AN ELONGATE FLEXIBLE MEDICAL DEVICE

(30) Priorité: 12.06.2014 FR 1455330
(43) Date de publication de la demande: 19.04.2017
(73) Titulaire: Robocath, 76000 Rouen (FR)
(72) Inventeur: DEBOEUF, Sébastien, 76300 Sotteville-Les-Rouen (FR); FOURNIER, Bruno, 41100 Saint Ouen (FR); MARIGNIER, Jacques, 76240 Le Mesnil Esnard (FR); OUALI, Inès, 13090 Aix en Provence (FR); BENCTEUX, Philippe, 76160 Saint Martin du Vivier (FR); DESTREBECQ, Fabien, 27520 Bourgtheroulde (FR)
(74) Mandataire: Plasseraud IP
(86) Numéro de dépôt international: PCT/FR2015/051566
(87) Numéro de publication internationale: WO 2015/189531

(56) Documents cités:
- EP-A1- 2 567 670
- WO-A1-2008/115151
- US-A1- 2007 185 404
- US-A1- 2009 082 722
- US-A1- 2012 179 167

## Description

La présente invention est relative aux modules robotisés d'entraînement d'organes médicaux souples allongés.

L'insertion manuelle d'un cathéter ou d'un guide dans un patient est un acte chirurgical relativement classique. Toutefois, cet acte étant monitoré sous rayons X, le chirurgien en charge de cet acte est soumis à une irradiation conséquente s'il réalise une telle opération sur de nombreux patients.

Afin de réduire les risques pour le chirurgien, on tente de robotiser une telle insertion. Cette robotisation est complexe, car la préhension du cathéter est difficile. Celui-ci baigne en effet dans du liquide de conservation et doit rester stérile. Par ailleurs, on veut pouvoir commander des mouvements de translation et de rotation alternatifs et/ou simultanés du cathéter. La fiabilité de ces systèmes robotisés est un critère déterminant.

Récemment, il a été proposé dans US 7,927,310 un système d'entraînement gérant à la fois la translation et la rotation du cathéter. Le cathéter est maintenu sur une plaquette rotative par rapport à une base pour l'entraînement en rotation. La plaquette elle-même comporte un mécanisme d'entraînement en translation. De plus, on fait appel à des moteurs déportés, à demeure sur le bâti, et à des systèmes de transfert du mouvement jusqu'au cathéter. En effet, on préfère ne pas avoir les moteurs embarqués, pour des raisons d'alimentation en puissance, d'encombrement et de stérilité. Les documents US 2009/082722 A1, US 2012/179167 A1, EP 2 567 670 A1, WO 2008/115151 A1 et US 2007/185404 A1 sont également des documents connus de l'état de la technique. Bien que cette configuration apporte toute satisfaction, on cherche toujours à simplifier ce genre de mécanismes en vue notamment d'en augmenter la fiabilité.

A cet effet, selon l'invention, on prévoit un module robotisé d'entraînement d'organe médical souple allongé comprenant les caractéristiques divulgué dans la revendication 1.

De préférence, l'organe de commande est déporté. Ainsi, il peut y avoir un seul organe de commande qui sera dans la salle de commande, l'organe de commande prévu dans la salle d'opérations étant parfois facultatif et donc pouvant être alors supprimé.

Grâce à ces dispositions, l'entraînement de l'organe médical souple allongé, même sur des trajectoires longues et complexes, peut être mis en œuvre par une répétition de déplacements élémentaires simples à l'échelle du robot. Le fonctionnement du robot est donc possible quelle que soit la trajectoire à faire parcourir au cathéter.

Dans des modes de réalisation préférés de l'invention, on peut éventuellement avoir recours en outre à l'une et/ou à l'autre des dispositions suivantes :
- le déplacement par rapport à la base des organes d'entraînement de la première position à la deuxième position comprend une combinaison de :
   - une translation des organes d'entraînement par rapport à la base selon une direction parallèle à la direction longitudinale locale de l'organe médical souple allongé,
   - une translation des organes d'entraînement par rapport à la base selon une direction transverse à la direction longitudinale locale de l'organe médical souple allongé et en des sens opposés,
   - une translation des organes d'entraînement par rapport à la base selon une direction transverse à la direction longitudinale locale de l'organe médical souple allongé et en un même sens,
   - une translation des organes d'entraînement par rapport à la base selon une direction transversale à la direction longitudinale locale de l'organe médical souple allongé et en un même sens ;
- un, deux ou trois membres de la combinaison sont nuls ;
- le degré de liberté entre les première et deuxième positions est un premier degré de liberté, dans lequel la paire d'organes d'entraînement est également montée mobile par rapport à la base selon un deuxième degré de liberté différant du premier degré de liberté entre la première et une troisième positions,
   l'organe de commande étant adapté pour commander de manière répétée cyclique un déplacement par rapport à la base (132) des organes d'entraînement en configuration d'entraînement de la première à la troisième position, entraînant ainsi l'organe médical souple allongé par rapport à la base, et un déplacement par rapport à la base des organes d'entraînement en configuration libre de la troisième à la première position sans entraîner l'organe médical souple par rapport à la base ;
- le déplacement par rapport à la base des organes d'entraînement de la première position à la deuxième position et le déplacement par rapport à la base des organes d'entraînement de la première à la troisième position comprennent deux combinaisons distinctes parmi :
   - une translation des organes d'entraînement par rapport à la base selon une direction parallèle à la direction longitudinale locale de l'organe médical souple allongé,
   - une translation des organes d'entraînement par rapport à la base selon une direction transversale à la direction longitudinale locale de l'organe médical souple allongé et en des sens opposés,
   - une translation des organes d'entraînement par rapport à la base selon une direction transverse à la direction longitudinale locale de l'organe médical souple allongé et en un même sens,
   - une translation des organes d'entraînement par rapport à la base selon une direction transverse à la direction longitudinale locale de l'organe médical souple allongé et en un même sens ;
- la translation des organes d'entraînement par rapport à la base selon une direction transverse à la direction longitudinale locale de l'organe médical souple allongé et en des sens opposés est adaptée pour permettre un roulement de l'organe médical souple allongé sur les surfaces d'entraînement autour de la direction longitudinale locale de l'organe médical souple allongé ;
- la paire d'organes d'entraînement est plaçable de sa configuration libre à sa configuration d'entraînement par un déplacement relatif des organes d'entraînement par rapport à la base ;
- les première et deuxième positions définissent un premier degré de liberté, dans lequel la paire d'organes d'entraînement est également montée mobile par rapport à la base selon un troisième degré de liberté de sa configuration libre à sa configuration d'entraînement ;
- la base est une première base, la paire d'organes d'entraînement est une première paire d'organes d'entraînement, le module robotisé comprenant en outre :
   - une deuxième base,
   - une deuxième paire d'organes d'entraînement présentant chacun une surface d'entraînement, la deuxième paire d'organes d'entraînement étant plaçable alternativement dans une configuration d'entraînement dans laquelle les surfaces d'entraînement des organes d'entraînement de la deuxième paire d'organes d'entraînement sont en prise avec l'organe médical souple allongé à entraîner et disposés de part et d'autre de celui-ci, et dans une configuration libre dans laquelle la surface d'entraînement des organes d'entraînement de la deuxième paire d'organes d'entraînement n'est pas en prise avec l'organe médical souple allongé,
      la deuxième paire d'organes d'entraînement étant montée mobile par rapport à la deuxième base selon un degré de liberté entre une première et une deuxième positions,
   - l'organe de commande étant adapté en outre pour commander de manière répétée cyclique un déplacement par rapport à la base des organes d'entraînement de la deuxième paire d'organes d'entraînement en configuration d'entraînement de la première à la deuxième position,
   entraînant ainsi l'organe médical souple allongé par rapport à la deuxième base, et un déplacement par rapport à la deuxième base des organes d'entraînement de la deuxième paire d'organes d'entraînement en configuration libre de la deuxième à la première position sans entraîner l'organe médical souple par rapport à la deuxième base ;
- la première base et la deuxième base sont solidaires ou communes ;
- l'organe de commande est adapté pour commander les déplacements des organes d'entraînement de la première paire et de la deuxième paire de manière synchronisée ;
- l'organe de commande est adapté pour placer les organes d'entraînement de la première paire et de la deuxième paire simultanément en configuration d'entraînement ;
- l'organe de commande est adapté pour placer les organes d'entraînement de la première paire et de la deuxième paire simultanément en configuration libre ;
- l'organe de commande est adapté pour placer simultanément les organes d'entraînement de la première paire et de la deuxième paire pour les uns en configuration d'entraînement et pour les autres en configuration libre ;
- la deuxième paire d'organes d'entraînement présente certaines caractéristiques décrites ci-dessus ;

Selon un autre aspect, l'invention se rapporte à un robot d'artériographie comprenant un récipient, un organe médical souple allongé au moins partiellement contenu dans le récipient, un module robotisé d'entraînement fixé au récipient, et adapté pour l'entraînement de l'organe médical souple allongé hors du récipient.

Par ailleurs, il a été proposé dans US 7,927,310 un système d'entraînement gérant à la fois la translation et la rotation du cathéter. Le cathéter est maintenu sur une plaquette rotative par rapport à une base pour l'entraînement en rotation. La plaquette elle-même comporte un mécanisme d'entraînement en translation. De plus, on fait appel à des moteurs déportés, à demeure sur le bâti, et à des systèmes de transfert du mouvement jusqu'au cathéter. En effet, on préfère ne pas avoir les moteurs embarqués, pour des raisons d'alimentation en puissance, d'encombrement et de stérilité.

Cette configuration propose donc un premier mode de fonctionnement dans lequel le cathéter et le guide de cathéter vont pouvoir avancer en translation.

Cette configuration propose donc également un deuxième mode de fonctionnement dans lequel le cathéter et le guide de cathéter vont pouvoir tourner sur eux-mêmes dans un même sens, le sens de rotation choisi pouvant être soit le sens horaire soit le sens antihoraire.

Cependant, dans certains passages du système de circulation sanguine du corps humain, comme par exemple un embranchement de veines ou d'artères ou bien comme par exemple une lésion, le passage du cathéter et a fortiori le passage du guide du cathéter qui le précède vont être difficiles et risquent de buter contre une paroi de vaisseau sanguin voire de s'accrocher et d'endommager une paroi de vaisseau sanguin ou encore de prendre un mauvais vaisseau sanguin au niveau d'un embranchement.

Pour pallier cette difficulté, un mode de réalisation de l'invention propose l'ajout d'un troisième mode de fonctionnement dans lequel une translation lente est associée à une rotation alternée rapide du guide de cathéter pour lui permettre de passer la zone sensible sans encombre. Cette translation lente associée à une rotation alternée rapide peut être effectuée pour le guide seul, pour le cathéter seul, pour le guide et le cathéter. Même si le guide et le cathéter sont entraînés, il est possible de n'appliquer ce troisième mode de fonctionnement qu'au guide ou qu'au cathéter. Cet aspect de l'invention pourra être revendiqué séparément.

A cet effet, on prévoit un procédé exemplaire robotisé d'entraînement de cathéter ou de guide, ou bien de cathéter et de guide, pilotant un ensemble d'organes d'entraînement et comprenant :
- un premier mode de fonctionnement dans lequel l'ensemble d'organes d'entraînement fait avancer en translation le guide et/ou le cathéter,
- un deuxième mode de fonctionnement dans lequel l'ensemble d'organes d'entraînement fait tourner le guide et/ou le cathéter autour de lui-même,
caractérisé en ce que le procédé comprend également :
- un troisième mode de fonctionnement dans lequel l'ensemble d'organes d'entraînement fait, simultanément, avancer en translation le guide et/ou le cathéter et tourner le guide et/ou le cathéter autour de lui-même alternativement dans un sens puis dans l'autre sens.

A cet effet, selon l'invention on prévoit aussi un module robotisé d'entraînement de cathéter ou de guide, ou bien de cathéter et de guide, comprenant un ensemble d'organes d'entraînement structuré et disposé de façon à pouvoir être piloté de manière à réaliser le procédé selon l'une quelconque des revendications précédentes.

A cet effet, selon l'invention on prévoit encore un module robotisé d'entraînement de cathéter ou de guide, ou bien de cathéter et de guide, comprenant un ensemble d'organes d'entraînement structuré et disposé de façon à pouvoir être piloté :
- dans un premier mode de fonctionnement, de manière à faire avancer en translation le guide et/ou le cathéter,
- dans un deuxième mode de fonctionnement, de manière à faire tourner le guide et/ou le cathéter autour de lui-même,
caractérisé en ce que l'ensemble d'organes d'entraînement est également structuré et disposé de façon à pouvoir être piloté :
- dans un troisième mode de fonctionnement, de manière à simultanément faire avancer en translation le guide et/ou le cathéter tout en le faisant tourner autour de lui-même alternativement dans un sens puis dans l'autre sens.

Dans des modes de réalisation préférés de l'invention, on peut éventuellement avoir recours en outre à l'une et/ou à l'autre des dispositions suivantes.

De préférence, dans le troisième mode de fonctionnement, l'ensemble d'organes d'entraînement fait, simultanément, avancer en translation le guide et/ou le cathéter en fonction des variations de la commande d'une interface homme machine et automatiquement tourner le guide et/ou le cathéter autour de lui-même alternativement dans un sens puis dans l'autre sens. Ainsi, l'ergonomie pour le praticien est améliorée tout en offrant une bonne efficacité de progression du guide de cathéter, et ceci sans danger d'accrochage de paroi de vaisseau sanguin pour le patient.

De préférence, dans le troisième mode de fonctionnement, l'ensemble d'organes d'entraînement fait, simultanément, avancer en translation le guide et/ou le cathéter en fonction des variations de la commande d'une interface homme machine et automatiquement tourner le guide et/ou le cathéter autour de lui-même alternativement dans un sens puis dans l'autre sens, la fréquence de rotation alternée étant proportionnelle à la vitesse de translation.

Dans un premier mode de réalisation préférentiel, pour lequel le praticien conserve une liberté maximale, il est prévu, un procédé robotisé d'entraînement de cathéter ou de guide, ou bien de cathéter et de guide, pilotant un ensemble d'organes d'entraînement et comprenant :
- un premier mode de fonctionnement dans lequel l'ensemble d'organes d'entraînement fait avancer en translation le guide et/ou le cathéter en fonction des variations de la commande d'une interface homme machine,
- un deuxième mode de fonctionnement dans lequel l'ensemble d'organes d'entraînement fait tourner le guide et/ou le cathéter autour de lui-même en fonction des variations de la commande d'une interface homme machine, caractérisé en ce que le procédé comprend également :
- un troisième mode de fonctionnement dans lequel l'ensemble d'organes d'entraînement fait, simultanément, avancer en translation le guide et/ou le cathéter et tourner le guide et/ou le cathéter autour de lui-même alternativement dans un sens puis dans l'autre sens, en fonction des variations de la commande d'une interface homme machine.

Dans un deuxième mode de réalisation préférentiel, pour lequel le praticien dispose d'une facilité d'utilisation optimisée, il est prévu, un procédé robotisé d'entraînement de cathéter ou de guide, ou bien de cathéter et de guide, pilotant un ensemble d'organes d'entraînement et comprenant :
- un premier mode de fonctionnement dans lequel l'ensemble d'organes d'entraînement fait automatiquement avancer en translation le guide et/ou le cathéter,
- un deuxième mode de fonctionnement dans lequel l'ensemble d'organes d'entraînement fait automatiquement tourner le guide et/ou le cathéter autour de lui-même, caractérisé en ce que le procédé comprend également :
- un troisième mode de fonctionnement dans lequel l'ensemble d'organes d'entraînement fait, automatiquement et simultanément, avancer en translation le guide et/ou le cathéter et tourner le guide et/ou le cathéter autour de lui-même alternativement dans un sens puis dans l'autre sens.

De préférence, dans le troisième mode de fonctionnement, l'ensemble d'organes d'entraînement fait, simultanément, avancer en translation le guide et/ou le cathéter et tourner le guide et/ou le cathéter autour de lui-même alternativement dans un sens puis dans l'autre sens, le rapport entre la fréquence de rotation alternée et la vitesse de translation étant réglable par l'utilisateur du procédé. Cela permet d'adapter, au souhait et à l'habileté de l'utilisateur, le rapport entre d'une part la fréquence de rotation alternée et d'autre part la vitesse de translation, tout en laissant éventuellement l'utilisateur avancer à son rythme selon les difficultés rencontrées avec la vitesse de translation que cet utilisateur va juger utile.

De préférence, dans le troisième mode de fonctionnement, l'avancée en translation du guide et/ou du cathéter est plus lente que dans le premier mode de fonctionnement, tandis que la rotation alternative du guide et/ou du cathéter autour de lui-même est plus rapide que la rotation du guide et/ou du cathéter autour de lui-même dans le deuxième mode de fonctionnement. Ainsi, la lenteur accentuée de la translation couplée avec l'augmentation de fréquence de la rotation alternée rend plus efficace le passage des zones sensibles, même si c'est au prix d'une dépense supplémentaire d'énergie par millimètre parcouru par le guide du cathéter.

De préférence, le guide est un fil présentant un bout recourbé, le bout recourbé avançant le long d'une direction parallèle au fil tout en tournant autour de l'axe du fil dans le troisième mode de fonctionnement. Ainsi, le bout recourbé du fil aide à diriger le guide du cathéter dans la bonne direction grâce à une orientation adéquate de ce bout recourbé du fil.

De préférence, le bout recourbé du guide subit au moins deux changements de sens de rotation le temps qu'il avance d'une distance correspondant à la longueur du bout recourbé, de préférence au moins quatre changements de sens de rotation, encore plus de préférence au moins dix changements de sens de rotation. Ainsi, la lenteur accentuée de la translation couplée avec l'augmentation de fréquence de la rotation alternée rend plus efficace le passage des zones sensibles, même si c'est au prix d'une dépense supplémentaire d'énergie par millimètre parcouru par le guide du cathéter.

De préférence, le guide et/ou le cathéter subit au moins deux changements de sens de rotation le temps qu'il avance d'une distance correspondant à une longueur de 5mm, de préférence au moins quatre changements de sens de rotation, encore plus de préférence au moins dix changements de sens de rotation. Ainsi, la lenteur accentuée de la translation couplée avec l'augmentation de fréquence de la rotation alternée rend plus efficace le passage des zones sensibles, même si c'est au prix d'une dépense supplémentaire d'énergie par millimètre parcouru par le guide du cathéter.

De préférence, dans le troisième mode de fonctionnement, la fréquence de changement de sens de rotation du guide et/ou du cathéter est d'au moins 1 Hz, de préférence d'au moins 3 Hz, encore plus de préférence d'au moins 10 Hz.

De préférence, dans le troisième mode de fonctionnement, la vitesse de translation du guide et/ou du cathéter est d'au plus 10 mm/s, de préférence d'au plus 3 mm/s, encore plus de préférence d'au plus 1 mm/s.

De préférence, le troisième mode est utilisé pour traverser certaines zones d'embranchement dans le système de circulation sanguine du corps humain. Ce troisième mode de fonctionnement est en effet particulièrement efficace pour traverser les zones sensibles ou difficiles du système de circulation sanguine du corps humain.

De préférence, le troisième mode est utilisé pour traverser certaines zones de lésion dans le système de circulation sanguine du corps humain. Ce troisième mode de fonctionnement est en effet particulièrement efficace pour traverser les zones sensibles ou difficiles du système de circulation sanguine du corps humain.

Selon un autre aspect de l'invention, toujours pour aider le guide et/ou le cathéter à passer les zones sensibles, il est prévu un procédé robotisé d'entraînement de cathéter ou de guide, ou bien de cathéter et de guide, pilotant un ensemble d'organes d'entraînement et comprenant :
- un premier mode de fonctionnement dans lequel l'ensemble d'organes d'entraînement fait avancer en translation le guide et/ou le cathéter,
- un deuxième mode de fonctionnement dans lequel l'ensemble d'organes d'entraînement fait tourner le guide et/ou le cathéter autour de lui-même,
caractérisé en ce que le procédé comprend également :
- un troisième mode de fonctionnement dans lequel l'ensemble d'organes d'entraînement fait, simultanément, d'une part avancer en translation le guide et/ou le cathéter et d'autre part faire successivement alterner une rotation du guide et/ou du cathéter autour de lui-même toujours dans un même sens suivie d'un arrêt de cette rotation.

De préférence, dans le troisième mode de fonctionnement, ladite rotation dure moins longtemps que ledit arrêt.

De préférence, dans le troisième mode de fonctionnement, ladite rotation dure entre 0.05s et 0.2s, de préférence environ 0.1s, ledit arrêt dure entre 0.3s et 1s, de préférence environ 0.5s, ladite vitesse de translation est comprise entre 1mm/s et 5mm/s, valant de préférence environ 3mm/s.

Dans des modules robotisés de l'art antérieur, que ce soit dans le domaine médical des cathéters ou dans d'autres domaines, les actionneurs, qui transmettent leur mouvement à l'organe d'entraînement, le transmettent par l'intermédiaire d'interfaces respectives entre les actionneurs respectifs et le socle de l'organe d'entraînement.

Or, dans ces modules robotisés de l'art antérieur, les interfaces sont situées à l'extérieur du socle de l'organe d'entraînement ou en région périphérique du socle de l'organe d'entraînement.

La structure d'implémentation de ces interfaces entre actionneurs et socle d'organe d'entraînement est alors relativement simple.

Un mode de réalisation de l'invention a toutefois détecté un problème de fiabilité de la transmission du mouvement entre actionneurs et socle d'organe d'entraînement dans ce cas-là.

En effet, ce mode de réalisation de l'invention a mis en évidence que ce problème de fiabilité vient du caractère décentré de la position des interfaces, entraînant alors une transmission déséquilibrée de l'effort.

Par ailleurs, chaque actionneur ne supporte que l'effort dans sa direction, il n'a pas à porter un ou plusieurs autre actionneurs dans une ou plusieurs autres directions, comme ce pourrait être le cas dans des systèmes existants : l'encombrement et le poids en sont nettement réduits.

C'est pourquoi, ce mode de réalisation de l'invention propose de disposer les interfaces de manière à ce que leur intersection soit située en région centrale du socle de l'organe d'entraînement, voire préférentiellement au centre de gravité du socle de l'organe d'entraînement, permettant alors une transmission équilibrée de l'effort, entraînant une transmission fiable du mouvement entre actionneurs d'une part et socle d'organe d'entraînement d'autre part. Cet aspect de l'invention pourra être revendiqué séparément.

Cela implique de faire entrer les interfaces à l'intérieur du socle de l'organe d'entraînement, ce qui rend la structure relativement plus complexe, mais nettement plus fiable au niveau de la qualité de transmission du mouvement entre actionneurs d'une part et socle d'organe d'entraînement d'autre part.

Le socle de l'organe d'entraînement est solidaire de l'organe d'entraînement et fixe par rapport à l'organe d'entraînement.

A cet effet, selon ce mode de réalisation de l'invention, on prévoit une chaîne de transmission de mouvement comprenant :
- un socle d'organe d'entraînement d'un élément mobile,
- trois actionneurs pilotant le socle de l'organe d'entraînement respectivement selon trois directions de translation distinctes entre elles, par l'intermédiaire de trois interfaces respectives avec le socle de l'organe d'entraînement,
caractérisée en ce que l'intersection des surfaces moyennes des trois interfaces est localisée en région centrale du socle de l'organe d'entraînement.

A cet effet, selon ce mode de réalisation de l'invention, on prévoit aussi une chaîne de transmission de mouvement comprenant :
- un socle d'un organe d'entraînement d'un élément mobile,
- trois actionneurs pilotant le socle de l'organe d'entraînement respectivement selon trois directions de translation distinctes entre elles, par l'intermédiaire de trois interfaces respectives avec le socle de l'organe d'entraînement,
caractérisée en ce que les trois interfaces sont sensiblement planes,
en ce que ces trois interfaces sont orthogonales les unes aux autres,
et en ce que ces trois interfaces sont imbriquées les unes dans les autres.

Dans des réalisations préférées de ce mode de réalisation de l'invention, on peut éventuellement avoir recours en outre à l'une et/ou à l'autre des dispositions suivantes.

De préférence, les trois directions de translation sont orthogonales les unes aux autres.

De préférence, les trois interfaces sont sensiblement planes, ces trois interfaces sont orthogonales les unes aux autres, et ces trois interfaces sont imbriquées les unes dans les autres. Ainsi, les trois interfaces peuvent être concentrées de manière relativement simple et vraiment efficace en région centrale du socle de l'organe d'entraînement.

De préférence, les trois interfaces sont des plaques de pression transmettant les poussées respectives des trois actionneurs. Ces plaques de forme plane permettent une transmission efficace des poussées des actionneurs pour un encombrement global relativement réduit.

De préférence, la première plaque comprend deux ouvertures orthogonales entre elles qui sont respectivement traversées par la deuxième plaque et par la troisième plaque, la deuxième plaque comprend une ouverture qui est traversée par la troisième plaque, l'ouverture de la deuxième plaque étant orthogonale aux deux ouvertures de la première plaque, la troisième plaque n'est traversée ni par la première plaque ni par la deuxième plaque. Cette façon d'imbriquer les plaques entre elles est relativement simple structurellement tout en restant efficace.

De préférence, chacune des ouvertures autorise un débattement de la plaque qui la traverse, ce débattement correspondant à la course de l'actionneur de la plaque qui traverse ladite ouverture, ce débattement étant supérieur à l'épaisseur de la plaque qui traverse ladite ouverture. En effet, si l'un des actionneurs bouge, le socle de l'organe d'entraînement ne doit bouger que dans la direction correspondant à cet actionneur ayant bougé et non pas dans les deux directions correspondant aux actionneurs restés immobiles. Pour cela, la présence de ces débattements permet de désolidariser les unes des autres les transmissions d'effort en provenance de chacun des différents actionneurs.

De préférence, chaque plaque est mobile en translation selon une direction parallèle à la droite constituée par l'intersection des deux autres plaques. Ainsi, la transmission des efforts deux à deux orthogonaux entre les actionneurs, est maintenue aisément.

De préférence, chaque plaque est reliée à son actionneur par deux barres symétriques par rapport à l'axe de poussée dudit actionneur, de préférence par quatre barres symétriques par rapport à l'axe de poussée dudit actionneur. Ainsi, la transmission d'effort en provenance de l'actionneur est bien répartie sur la plaque correspondante.

De préférence, le socle de l'organe d'entraînement est solidarisé de manière fixe à chacune des interfaces de manière à ce que le déplacement de l'une des interfaces entraîne automatiquement le même déplacement du socle de l'organe d'entraînement. Ainsi, la transmission d'effort entre d'une part les interfaces et d'autre part le socle d'organe d'entraînement est plus directe.

De préférence, le socle de l'organe d'entraînement est un cube à l'intérieur duquel sont situées les trois interfaces. Ainsi, le volume global du socle de l'organe d'entraînement est relativement réduit, tandis que les interfaces sont toutefois totalement incluses dans le socle de l'organe d'entraînement. La compacité globale s'en trouve par conséquent améliorée.

De préférence, le socle de l'organe d'entraînement est un cube résultant de l'assemblage de huit cubes plus petits assemblés autour des interfaces. Ces huit petits cubes représentent le nombre minimal de sous parties du cube constituant le socle de l'organe d'entraînement, de manière à pouvoir assembler ce cube autour de l'ensemble des trois interfaces imbriquées entre elles.

De préférence, chaque plaque est coincée entre quatre cubes plus petits d'un côté et quatre cubes plus petits de l'autre côté. Le socle d'organe d'entraînement est ainsi complètement symétrique et équilibré.

De préférence, la région centrale est le centre de gravité du socle de l'organe d'entraînement. La transmission des efforts entre actionneurs et socle d'organe d'entraînement est ainsi parfaitement équilibrée grâce au caractère alors parfaitement centré des interfaces par rapport au socle de l'organe d'entraînement.

De préférence, la chaîne de transmission de mouvement comprend un élément mobile entraîné par l'organe d'entraînement.

Préférentiellement, les matériaux utilisés sont des matériaux à faible voire à très faible friction, pour permettre aux interfaces imbriquées les unes dans les autres de coulisser aisément.

Dans une application préférentielle mais non exclusive, l'élément mobile est un cathéter ou un guide de cathéter, l'organe d'entraînement est un organe de serrage d'un cathéter ou d'un guide de cathéter.

Dans une famille de modes de réalisation, le mouvement des actionneurs est transmis à une paire d'organes d'entraînement par une pièce intermédiaire. L'actionneur pilote une paire d'organes d'entraînement. La pièce intermédiaire transmet le déplacement de l'actionneur à la paire d'organes d'entraînement, de manière à translater dans des sens opposés les deux organes d'entraînement de la paire d'organes d'entraînement, tout en maintenant sensiblement constant l'écartement entre les deux organes d'entraînement de la paire d'organes d'entraînement, de façon à faire tourner un organe médical souple allongé autour de lui-même lorsque celui-ci est disposé entre les deux organes d'entraînement de la paire d'organes d'entraînement. La présence de cette pièce intermédiaire permet une plus grande précision, dans la mesure où un mouvement d'amplitude importante de l'actionneur est traduit par un mouvement d'amplitude limitée des organes d'entraînement. Cet aspect de l'invention pourra être revendiqué séparément.

De préférence, la pièce intermédiaire est une bascule transformant une translation de l'actionneur selon une première direction en deux translations de sens opposés des deux organes respectifs d'entraînement selon une deuxième direction orthogonale à la première direction. Ce renvoi directionnel facilite la réalisation de la réduction de l'amplitude du mouvement transmis.

Dans une première réalisation, la bascule comprend une platine qui est reliée à l'actionneur et qui présente deux trous oblongs inclinés d'inclinaison contraire dans lesquels coulissent au moins deux pions respectivement reliés aux organes d'entraînement, l'inclinaison des trous oblongs étant plus proche de la première direction que de la deuxième direction. Cette première réalisation présente l'avantage de la simplicité structurelle.

Dans une deuxième réalisation, la bascule comprend une platine qui est reliée à l'actionneur et qui présente deux trous oblongs inclinés d'inclinaison contraire dans lesquels coulissent au moins deux galets respectivement reliés aux organes d'entraînement, l'inclinaison des trous oblongs étant plus proche de la première direction que de la deuxième direction. Cette deuxième réalisation présente l'avantage d'une moindre usure et d'une plus grande durée de vie, grâce à l'utilisation de galets au lieu de pions.

Dans une troisième réalisation, la bascule comprend une platine qui est reliée à l'actionneur et qui présente deux rails obliques inclinés d'inclinaison contraire dans lesquels coulissent au moins deux coulisseaux respectivement reliés aux organes d'entraînement, l'inclinaison des rails étant plus proche de la première direction que de la deuxième direction. Cette troisième réalisation présente l'avantage d'une meilleure robustesse en raison de la plus grande surface de contact entre rail et coulisseau.

Dans une première variante de la troisième réalisation, les deux rails sont dans un même plan parallèle au plan formé par la première direction et par la deuxième direction. L'encombrement est moindre.

Dans une deuxième variante de la troisième réalisation, les deux rails sont dans deux plans distincts perpendiculaires au plan formé par la première direction et par la deuxième direction. La robustesse est encore améliorée car le poids du coulisseau sur le rail s'exerce sur toute la surface du rail et non pas en porte-à-faux.

Dans une quatrième réalisation, la bascule est pivotante autour d'un axe et comprend une platine qui est reliée à l'actionneur et qui présente trois trous oblongs inclinés de même inclinaison dans lesquels coulissent au moins trois pions ou trois galets respectivement reliés à l'actionneur et aux organes d'entraînement, l'inclinaison des trous oblongs étant plus proche de la première direction que de la deuxième direction, deux des trous oblongs étant disposés de manière symétrique par rapport à l'axe pivotant et recevant les pions ou les galets reliés respectivement aux deux organes d'entraînement, le troisième trou oblong étant disposé plus loin de l'axe pivotant que les deux trous oblongs reliés aux organes d'entraînement et recevant le pion ou le galet relié à l'actionneur.

Dans une cinquième réalisation, la bascule comprend une platine qui est reliée à l'actionneur et qui présente deux systèmes à biellette et manivelle en forme de L, les deux manivelles en forme de L étant orientées en sens contraire, la petite partie du L des manivelles étant sensiblement selon la première direction, la grande partie du L des manivelles étant sensiblement selon la deuxième direction.

Dans une sixième réalisation, la bascule comprend une platine qui est reliée d'un côté à l'actionneur et qui est reliée de l'autre côté à une première extrémité de bielle dont la deuxième extrémité est reliée à une première extrémité d'une première tige coulissant en son milieu dans un premier trou oblong situé à une première extrémité d'une barre pivotant en son milieu et dont la deuxième extrémité présente un deuxième trou oblong dans lequel coulisse le milieu d'une deuxième tige, les trous oblongs étant parallèles à la barre, les deuxièmes extrémités des deux tiges étant respectivement reliées aux organes d'entraînement.

Dans une septième réalisation, la bascule comprend une platine qui est reliée à l'actionneur et qui présente une première crémaillère selon la première direction, deux deuxièmes crémaillères qui sont respectivement reliées aux organes d'entraînement et qui sont selon la deuxième direction et dont les parties dentées se font face, deux systèmes d'engrenages situés entre la première crémaillère et les deux deuxièmes crémaillères respectives, chacun des systèmes d'engrenage comprenant un grand engrenage engrenant avec la première crémaillère et un petit engrenage engrenant avec une des deuxièmes crémaillères. Cette quatrième réalisation présente l'avantage de la simplicité structurelle. Cette septième réalisation présente l'avantage d'un encombrement réduit.

Selon un autre aspect de l'invention, il est prévu que la transmission d'effort d'un actionneur sur le socle d'organe entraînement n'entraîne pas de mouvement perturbateur suivant la direction de cet actionneur pour le ou les autres actionneurs. Cet aspect pourra être revendiqué séparément. Cet aspect pourra également être revendiqué en combinaison avec tout autre aspect de l'invention présentée dans l'ensemble de ce texte.

Préférentiellement, il est prévu un module robotisé d'entraînement d'un organe médical souple allongé, caractérisé en ce qu'il comprend une chaîne de transmission de mouvement comprenant :
- un socle d'organe d'entraînement d'un organe médical souple allongé,
- deux actionneurs pilotant le socle de l'organe d'entraînement respectivement selon deux directions de translation distinctes entre elles, par l'intermédiaire de deux interfaces respectives avec le socle de l'organe d'entraînement,
et en ce que chacune des deux interfaces autorise un mouvement libre du socle par rapport à son actionneur dans la direction associée à l'autre interface.

Préférentiellement, il est aussi prévu un module robotisé d'entraînement d'un organe médical souple allongé, caractérisé en ce qu'il comprend une chaîne de transmission de mouvement comprenant :
- un socle d'organe d'entraînement d'un organe médical souple allongé,
- trois actionneurs pilotant le socle de l'organe d'entraînement respectivement selon trois directions de translation distinctes entre elles, par l'intermédiaire de trois interfaces respectives avec le socle de l'organe d'entraînement,
et en ce que chacune des trois interfaces autorise un mouvement libre du socle par rapport à son actionneur dans les deux directions respectivement associées aux autres interfaces.

Chaque actionneur ne supporte que l'effort dans sa direction, il n'a pas à porter un ou plusieurs autre actionneurs dans une ou plusieurs autres directions, comme ce pourrait être le cas dans des systèmes existants : l'encombrement et le poids en sont nettement réduits.

Le socle de l'organe d'entraînement est solidaire de l'organe d'entraînement et fixe par rapport à l'organe d'entraînement.

Par exemple, lorsque l'interface de l'actionneur de la direction X transmet un effort selon la direction X, le socle se déplace selon la direction X, librement par rapport aux actionneurs selon les directions Y et Z, donc sans gêner ou perturber ces actionneurs selon les directions Y et Z.

Par exemple, lorsque l'interface de l'actionneur de la direction Y transmet un effort selon la direction Y, le socle se déplace selon la direction Y, librement par rapport aux actionneurs selon les directions X et Z, donc sans gêner ou perturber ces actionneurs selon les directions X et Z.

Par exemple, lorsque l'interface de l'actionneur de la direction Z transmet un effort selon la direction Z, le socle se déplace selon la direction Z, librement par rapport aux actionneurs selon les directions Y et X, donc sans gêner ou perturber ces actionneurs selon les directions Y et X.

De préférence, les deux ou trois directions de translation sont orthogonales les unes aux autres.

De préférence, au moins l'une, de préférence au moins deux, encore plus de préférence trois, des deux ou trois interfaces est ou sont localisée(s) à l'intérieur du socle. Cela permet d'améliorer le centrage de la transmission d'effort du ou des actionneurs vers le socle, et ainsi d'améliorer la précision et la fiabilité du module robotisé.

De préférence, le socle est en forme de cube.

De préférence, le ou les matériaux des interfaces est ou sont à suffisamment faible friction pour que ledit mouvement libre soit totalement fluide.

Cet aspect pourra également être revendiqué séparément et indépendamment de l'organe médical souple allongé, c'est-à-dire pour tout type d'élément mobile.

Il est alors prévu un module robotisé d'entraînement d'un élément mobile, caractérisé en ce qu'il comprend une chaîne de transmission de mouvement comprenant :
- un socle d'organe d'entraînement d'un élément mobile,
- deux actionneurs pilotant le socle de l'organe d'entraînement respectivement selon deux directions de translation distinctes entre elles, par l'intermédiaire de deux interfaces respectives avec le socle de l'organe d'entraînement,
et en ce que chacune des deux interfaces autorise un mouvement libre du socle par rapport à son actionneur dans la direction associée à l'autre interface.

Il est alors aussi prévu un module robotisé d'entraînement d'un élément mobile, caractérisé en ce qu'il comprend une chaîne de transmission de mouvement comprenant :
- un socle d'organe d'entraînement d'un élément mobile,
- trois actionneurs pilotant le socle de l'organe d'entraînement respectivement selon trois directions de translation distinctes entre elles, par l'intermédiaire de trois interfaces respectives avec le socle de l'organe d'entraînement,
et en ce que chacune des trois interfaces autorise un mouvement libre du socle par rapport à son actionneur dans les deux directions respectivement associées aux autres interfaces.

Le socle de l'organe d'entraînement est solidaire de l'organe d'entraînement et fixe par rapport à l'organe d'entraînement.

De préférence, les deux ou trois directions de translation sont orthogonales les unes aux autres.

De préférence, au moins l'une, de préférence au moins deux, encore plus de préférence trois, des deux ou trois interfaces est ou sont localisée(s) à l'intérieur du socle.

De préférence, le socle est en forme de cube.

De préférence, le ou les matériaux des interfaces est ou sont à suffisamment faible friction pour que ledit mouvement libre soit totalement fluide.

D'autres caractéristiques et avantages de l'invention apparaîtront au cours de la description suivante d'une de ses formes de réalisation, donnée à titre d'exemple non limitatif, en regard des dessins joints.

Sur les dessins :
- la figure la est une vue schématique de côté d'une installation d'artériographie robotisée.

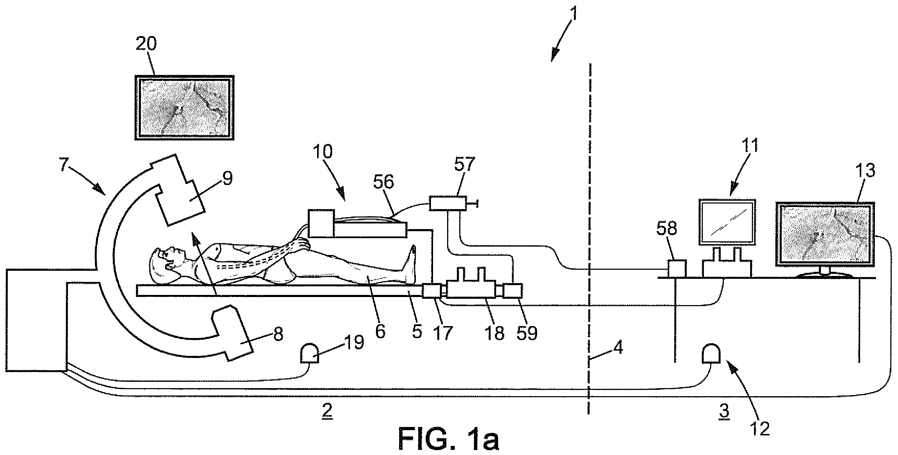

- la figure 1b est une vue de dessus d'une partie de la figure la,
- la figure 2 est une vue schématique de dessus d'un robot utilisé dans l'installation des figures la et 1b,
- les figures 3a-3c sont des schémas illustratifs des modes de déplacement des organes à entraîner,
- les figures 4 et 5 sont des schémas cinématiques simplifiés de l'actionnement d'un organe d'entraînement,
- la figure 6 est une vue schématique en perspective d'une portion d'un module d'entraînement en configuration libre,
- les figures 7a à 7e sont des schémas simplifiés illustrant un cycle d'entraînement en translation du cathéter selon un mode de réalisation,
- les figures 8a à 8e sont des schémas simplifiés illustrant un cycle d'entraînement en rotation du cathéter selon l'invention,
- les figures 9a à 9f sont des schémas simplifiés illustrant un cycle d'entraînement en translation du cathéter selon un mode de réalisation,
- la figure 10 est un schéma simplifié illustrant un mode manivelle,
- les figures 11-13 sont d'autres schémas illustratifs des modes de déplacement des organes à entraîner,
- la figure 14a est une vue en perspective d'un exemple de réalisation de système d'actionnement,
- la figure 14b est une vue de détail de la figure 14a,
- la figure 15 est un schéma cinématique d'un exemple d'entraînement en translation selon un exemple de réalisation,
- la figure 16a est une vue plane dans le plan X-Z d'un exemple d'actionneur,
- les figures 16b et 16c sont deux coupes dans des plans distincts transversaux au plan X-Z de l'exemple d'actionneur de la figure 16a,
- la figure 17 est une vue plane d'un système d'actionnement dans deux dimensions,
- les figures 18a à 18m sont des schémas simplifiés illustrant une portion d'un cycle d'entraînement en mode « manivelle » de l'organe médical souple allongé selon un mode de réalisation.
- la figure 19 est une vue en perspective d'un autre exemple de réalisation d'un cube transmettant le mouvement des trois actionneurs à l'organe d'entraînement.
- la figure 20 est une autre vue en perspective d'un autre exemple de réalisation d'un cube transmettant le mouvement des trois actionneurs à l'organe d'entraînement.
- la figure 21 est encore une autre vue en perspective d'un autre exemple de réalisation d'un cube transmettant le mouvement des trois actionneurs à l'organe d'entraînement. Cette figure est similaire à la figure précédente, mais cette fois-ci, l'ensemble est montré de façon montée, et non plus en vue éclatée.
- la figure 22 est une vue en perspective d'encore un autre exemple de réalisation d'un cube transmettant le mouvement des trois actionneurs à l'organe d'entraînement.
- la figure 23 est une autre vue en perspective d'encore un autre exemple de réalisation d'un cube transmettant le mouvement des trois actionneurs à l'organe d'entraînement.
- la figure 24 est une vue en perspective d'un exemple de réalisation de l'intersection et de l'imbrication des interfaces entre actionneurs d'une part et socle d'organe d'entraînement d'autre part.
- la figure 25 est une vue en perspective d'un exemple de réalisation du socle d'organe d'entraînement.
- la figure 26 est une vue en perspective d'un exemple de réalisation d'assemblage entre d'une part les interfaces et d'autre part le socle d'organe d'entraînement.
- la figure 27 est une autre vue en perspective d'un exemple de réalisation d'assemblage entre d'une part les interfaces et d'autre part le socle d'organe d'entraînement.
- les figures 28 à 35 représentent une pièce intermédiaire transmettant le mouvement d'un actionneur à la paire d'organes d'entraînement pilotée par cet actionneur.

Sur les différentes figures, les mêmes références désignent des éléments identiques ou similaires.

La figure la représente schématiquement une installation d'artériographie 1. L'installation d'artériographie 1 est divisée en deux endroits distincts, une salle d'opérations 2 et une salle de commande 3. La salle de commande 3 peut être proche de la salle d'opération 2, séparée de celle-ci par une simple paroi 4, opaque aux rayons X, ou distante. Les matériels de la salle d'opération 2 et de la salle de commande 3 sont reliés entre eux de manière fonctionnelle, par voie filaire, sans fil ou réseau, _{... .}

La salle d'opérations 2 comprend une table d'opérations 5 recevant un patient 6. La salle d'opérations 2 peut également comprendre un imageur médical 7, notamment un imageur par rayons X, comprenant une source 8 et un détecteur 9 disposés de part et d'autre du patient, éventuellement mobiles par rapport au patient.

L'installation d'artériographie 1 comprend un robot 10 disposé dans la salle d'opérations 2.

L'installation d'artériographie 1 comprend un poste de commande 11 disposé dans la salle de commande 3. Le poste de commande 11 est adapté pour commander à distance le robot 10. L'installation d'artériographie 1 peut également comprendre, disposée dans la salle de commande 3, une ou plusieurs commandes distantes 12 de l'imageur 7, communiquant avec l'imageur 7 pour commander celui-ci à distance. L'installation d'artériographie 1 peut également comprendre, disposé dans la salle de commande 3, un écran 13, communiquant avec l'imageur 7, pour visualiser en temps réel dans la salle de commande 3 les images acquises par l'imageur 7.

Le robot 10 peut comprendre un récipient 14 adapté pour contenir un organe médical souple allongé 15 à introduire dans le corps d'un patient. A titre d'organe médical souple allongé 15, il peut par exemple s'agir d'un organe à introduire dans un canal d'un patient, et à déplacer dans ce canal, notamment une artère ou une veine d'un patient, à travers un désilet ménageant une ouverture d'accès dans le patient. L'organe médical souple allongé peut être notamment un cathéter. En variante, l'organe médical souple allongé peut être un guide pour cathéter. Un guide est généralement de diamètre transversal inférieur à celui du cathéter, qui est généralement creux sur une portion proche du patient, voire sur la totalité de sa longueur, de sorte que le guide puisse se déplacer à l'intérieur de celui-ci, notamment à l'intérieur du corps du patient. Le guide peut également comporter une extrémité recourbée, comme il sera décrit plus en détail ci-après.

Le robot 10 peut comprendre un module d'entraînement 16 de l'organe médical souple allongé 15. Le module d'entraînement 16 est commandable à partir du poste de commande 11 pour entraîner l'organe médical souple allongé par rapport au patient selon au moins un degré de liberté, comme ce sera décrit en détails par la suite. Le module d'entraînement peut comprendre un boîtier de communication 17 servant à l'interfaçage avec le poste de commande 11. Au besoin, le robot 10 peut comprendre un boîtier de commande 18 en local, destiné à commander le robot depuis la salle d'opérations 2 si nécessaire.

On notera d'ailleurs que toutes les commandes et les retours disponibles dans la salle de commande 3 peuvent être également disponibles dans la salle d'opérations 2 en vue d'une opération en local, comme par exemple une commande 19 de l'imageur et un écran 20 permettant de visualiser les images acquises par l'imageur 7.

L'organe médical souple allongé 15 creux peut être raccordé à un raccord 56 permettant l'injection d'un produit de contraste facilitant l'imagerie à l'intérieur de l'organe médical souple allongé. L'installation d'artériographie peut comprendre un injecteur 57 de produit de contraste raccordé au raccord 56, commandable par une commande 58 disposée dans la salle de commande 3. Une commande 59 de l'injecteur de produit de contraste peut également être présente en local dans la salle d'opérations 2.

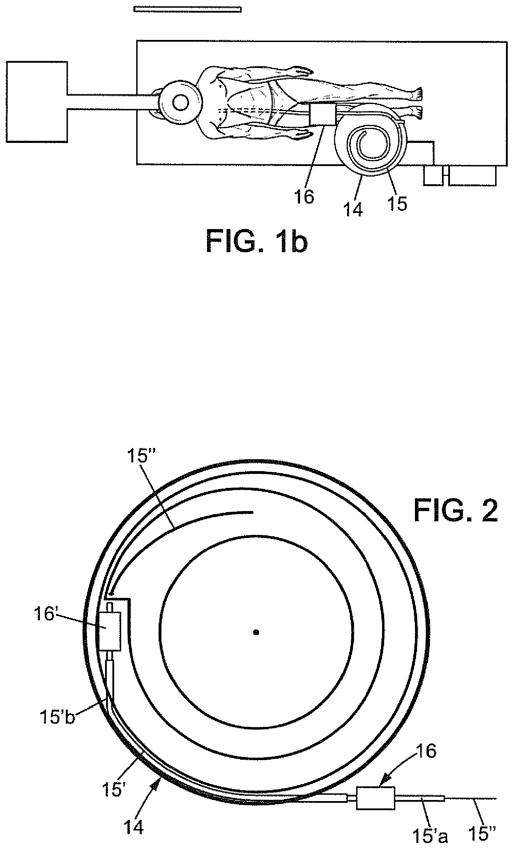

Comme visible sur la figure 2, de manière purement illustrative, on a représenté plus en détail le récipient 14 recevant un cathéter 15'. Le récipient 14 permet de maintenir le cathéter 15' dans un milieu convenant à sa conservation. Le module d'entraînement 16 est adapté pour l'entraînement du cathéter 15'. Dans l'exemple, on prévoit que le récipient 14 reçoit également un guide 15". Le récipient 14 permet de maintenir le guide 15" dans un milieu convenant à sa conservation. Le module d'entraînement 16' est adapté pour l'entraînement du guide 15". Selon les applications, les modules d'entraînement 16, 16', peuvent être identiques ou différents. Ils peuvent être selon l'un des modes de réalisation présentés ci-dessous, si approprié. Dans l'exemple présenté, le guide 15" peut être introduit dans le cathéter 15' à l'extrémité arrière 15'b de celui-ci, et dépasser de l'extrémité avant 15'a du cathéter comme représenté.

Dans ce qui suit, on utilisera la référence 15 pour désigner alternativement le guide 15", le cathéter 15', ou de manière générale un organe médical souple allongé à introduire dans le corps d'un patient. Il peut par exemple s'agir d'un cathéter interventionnel. Un tel cathéter interventionnel peut être de diamètre inférieur au cathéter, de manière à être guidé à l'intérieur de celui-ci, coaxialement à l'intérieur du patient, et être creux de manière à être guidé sur le guide à l'intérieur du patient.

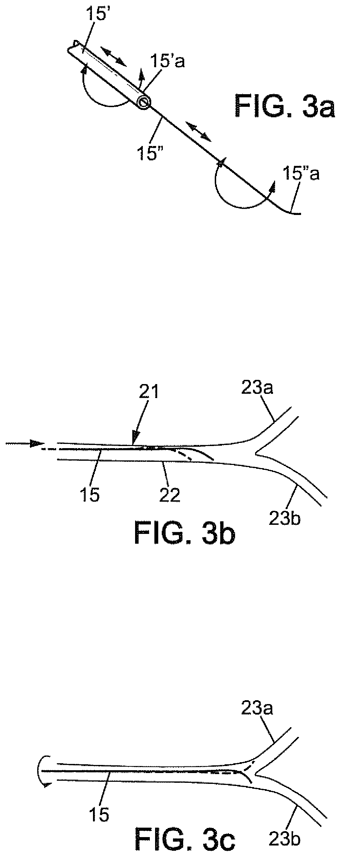

La figure 3a représente les divers degrés de liberté envisageables avec le présent système. On visualise le guide 15" avec son extrémité avant 15" a légèrement courbée par rapport à l'axe longitudinal principal du guide, et débouchant par l'extrémité avant 15'a du cathéter 15'. Le cathéter 15' peut être soumis à deux mouvements distincts :
- Une translation selon son axe longitudinal,
- Une rotation autour de son axe longitudinal.

Ces mouvements peuvent être générés dans un sens ou dans l'autre.

Le cas échéant, le cathéter 15' peut être soumis à un mouvement combiné des deux mouvements simples décrits ci-dessus.

Le cas échéant, le cathéter 15' peut être soumis à deux mouvements combinés des deux mouvements simples décrits ci-dessus, selon des combinaisons différentes.

Ce qui a été décrit ci-dessus concernant le cathéter s'applique également au guide.

Dans certains cas, le cathéter est lui-même pourvu d'une extrémité courbe, soit pour permettre la navigation sur le même principe qu'un guide, soit pour faciliter le positionnement dans une zone anatomique présentant une courbure particulière.

Sur la figure 3b, on a représenté une artère 21 d'un patient comprenant un tronc principal 22 et deux branches 23a, 23b débouchant sur le tronc principal. La figure 3b illustre le déplacement d'un organe médical souple allongé 15 (ici un guide 15") selon une translation entre une position reculée représentée en pointillés et une position avancée représentée en traits pleins. Sur la figure 3c, dans la même artère, on a représenté une rotation de l'organe médical souple allongé 15 entre une première position, représenté en pointillés, où l'organe médical souple allongé est prêt à être soumis à une translation en direction de la branche 23a, et une deuxième position, représentée en traits pleins, où l'organe médical souple allongé est prêt à être soumis à une translation en direction de la branche 23b.

L'organe médical souple allongé peut être entraîné selon le ou les déplacements décrits ci-dessus par des organes d'entraînement. Les organes d'entraînement peuvent être agencés par paires.

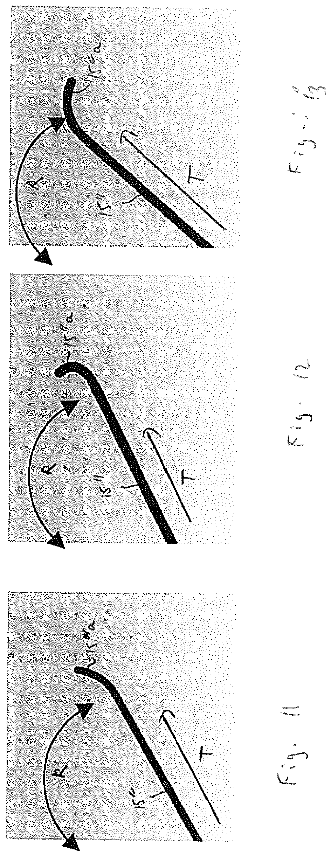

Sur les figures 11 à 13 ont été représentés des mouvements de translation et des mouvements de rotation correspondant aux premier et deuxième modes de fonctionnement.

Maintenant en liaison avec les figures 3d à 3f, va être présenté le troisième mode de fonctionnement.

Le guide 15" de cathéter et son bout recourbé 15"a progressent en translation T le long du guide 15", tandis que, simultanément, le guide 15" de cathéter et son bout recourbé 15"a subissent une rotation R alternée autour de l'axe du guide 15" de cathéter.

Les trois figures 3d, 3e et 3f, représentent le bout recourbé 15"a dans différentes positions de d'orientation angulaire lors de la rotation alternée R.

La vitesse de translation T est relativement lente, tandis que la fréquence de rotation alternée R est relativement élevée. Ce troisième mode de fonctionnement, de type translation lente avec rotation alternée rapide simultanée, permet au guide 15"a de cathéter de passer aisément les zones sensibles ou difficiles dans la circulation sanguine de corps humain. C'est le caractère rotation rapide sur une faible course de translation qui permet le passage de la zone délicate sans encombre et sans risque d'accrochage dans la paroi d'un vaisseau sanguin du patient.

Selon un exemple de réalisation, un organe d'entraînement donné peut être actionné par un actionneur.

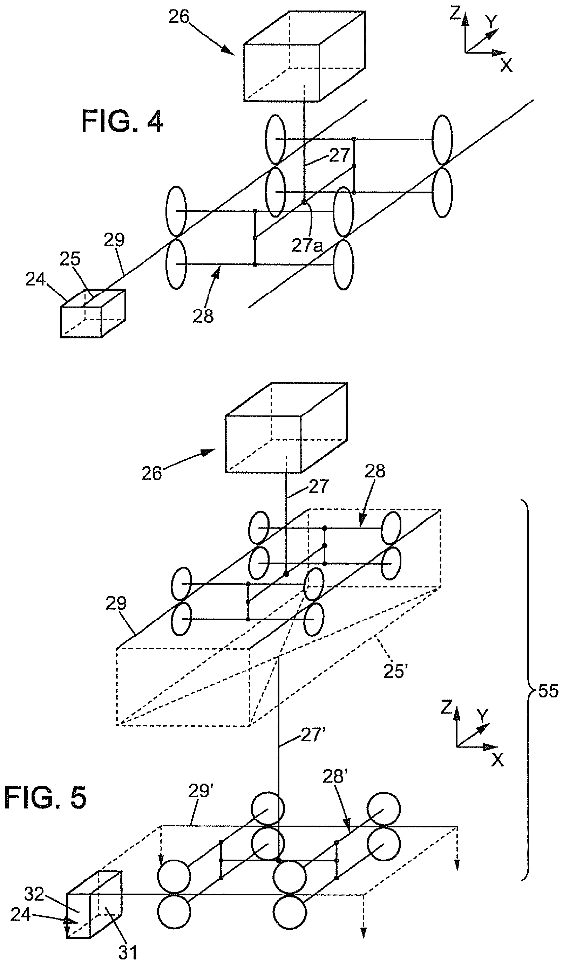

En se référant à la figure 4, on décrit un organe d'entraînement 24 actionné selon une unique direction Z. L'organe d'entraînement 24 comprend une surface d'actionnement 25 normale à l'axe Z. L'actionneur 26 comprend une tige d'actionnement 27 comprenant une extrémité 27a destinée à solliciter la surface d'actionnement 25 selon la direction Z. L'organe d'entraînement 24 est mobile librement selon la direction Y sans perte de contact avec la tige d'actionnement 27. L'extrémité 27a de la tige d'actionnement 27 glisse par rapport à la surface d'actionnement 25 selon la direction Y. On prévoit par exemple que la tige d'actionnement 27 est solidaire d'un charriot 28 monté coulissant selon la direction Y sur des rails 29 solidaires de l'organe d'entraînement 24.

Dans ces conditions, si on déplace la surface d'actionnement 25 de l'organe d'entraînement 24 selon la direction Y, on ne génère pas de déplacement du chariot 28, et par conséquent de l'actionneur 26. Si on déplace l'extrémité 27a de l'actionneur selon la direction Z vers le bas (sur la figure 4), on génère un déplacement de l'organe d'entraînement 24 selon la direction Z vers le bas. La plage de déplacement du charriot 28 par rapport à l'organe d'entraînement 24 selon la direction Y est de préférence limitée pour que, en chaque emplacement de cette plage de déplacement, l'extrémité 27a de la tige d'actionnement reste en prise avec la surface d'actionnement 25 de l'organe d'entraînement 24.

Pour un déplacement vers le bas sur la figure 4, il suffit que l'actionneur 26 s'allonge par rapport à sa référence fixe. Pour un déplacement vers le haut, il suffit qu'il se raccourcisse.

Comme visible sur la figure 5, le mécanisme qui vient d'être décrit peut être dupliqué pour permettre un déplacement libre de l'organe d'entraînement 24 dans tout le plan X-Y, et un actionnement selon la seule direction Z. Le mécanisme décrit sur la figure 4 correspond alors au premier étage d'un mécanisme décrit sur la figure 5, dont le deuxième étage permet d'entraîner l'organe d'entraînement selon la direction Z mais pas selon la direction X. Pour ce faire, on peut avoir recours à un charriot 28' mobile le long de rails 29' selon l'axe X par rapport à l'organe d'entraînement 24 et portant une tige de liaison 27' coopérant avec une surface intermédiaire liée aux rails 29.

Dans ces conditions :
- Un raccourcissement de l'actionneur 26 selon la direction Z cause un déplacement de l'organe d'entraînement 24 selon la direction Z vers le haut,
- Un allongement de l'actionneur 26 selon la direction Z permet un déplacement de l'organe d'entraînement 24 selon la direction Z vers le bas,
- Un déplacement de l'organe d'entraînement 24 selon la direction X ne cause aucun autre déplacement,
- Un déplacement de l'organe d'entraînement 24 selon la direction Y cause le déplacement du chariot 28' selon la direction Y par rapport au charriot 28.

Le système d'actionnement 55 décrit sur la figure 5 permet l'actionnement de l'organe d'entraînement 24 selon une unique direction Z tant que les charriots 28, 28' ne sortent pas des rails associés.

Le système d'actionnement 55 est par exemple à base d'actionneurs électromagnétiques ou piézo-électriques, par exemple.

On peut prévoir un système d'actionnement 55' similaire pour l'actionnement de l'organe d'entraînement 24 selon une unique direction X. Il suffit pour cela de faire tourner le système représenté à la figure 5 de 90° autour de l'axe Y, les rails 29' de ce système étant alors solidaires d'une surface d'actionnement 31 de l'organe d'entraînement 24 qui soit normale à l'axe X.

On peut prévoir un système d'actionnement similaire pour l'actionnement de l'organe d'entraînement 24 selon une unique direction Y. Il suffit pour cela de faire tourner le système représenté à la figure 5 de 90° autour de l'axe X, les rails 29' de ce système étant alors solidaires d'une surface d'actionnement 32 de l'organe d'entraînement 24 qui soit normale à l'axe Y.

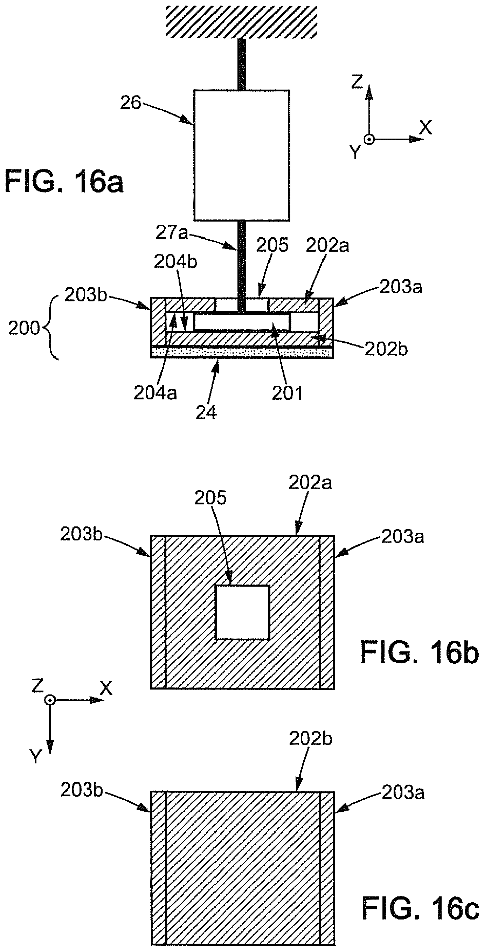

Dans un autre mode de réalisation, le principe de double charriot est remplacé par un ensemble 200 (voir figure 16) composé d'une pièce d'interface 201, par exemple de forme cylindrique, prise en sandwich entre deux plaques 202a et 202b, elles-mêmes maintenues solidaires l'une de l'autre, par exemple à l'aide de deux pièces 203a et 203b, la distance entre les deux surfaces intérieures 204a et 204b des plaques 202a et 202b étant sensiblement égale à l'épaisseur de la pièce d'interface 201. Afin de laisser passer l'axe 27 de l'actionneur 26, une ouverture, par exemple de forme sensiblement rectangulaire ou carrée 205, est pratiquée dans la plaque 202a. Une action de l'actionneur 26 suivant l'axe Z provoque alors le déplacement correspondant de la surface d'actionnement 25 de l'organe d'entraînement 24, solidaire de la plaque 202b suivant l'axe Z. Simultanément, l'ensemble 200 peut se déplacer librement suivant les axes X et Y, l'actionneur 26 restant immobile suivant ces deux axes. Pour autoriser ce mouvement de glissement relatif entre la pièce d'interface 201, d'une part, et l'ensemble 200, d'autre part, la pièce d'interface 201 doit pouvoir glisser avec le moins de frottement possible sur les surfaces 204a et 204b. On pourra utiliser pour cela des matériaux à très faible coefficient de frottement. Par exemple - mais ceci n'est pas limitatif - la pièce d'interface 201 pourra être réalisée en aluminium, et les pièces 202a et 202b dans un matériau plastique contenant du Polytétrafluoroéthylène (PTFE) .

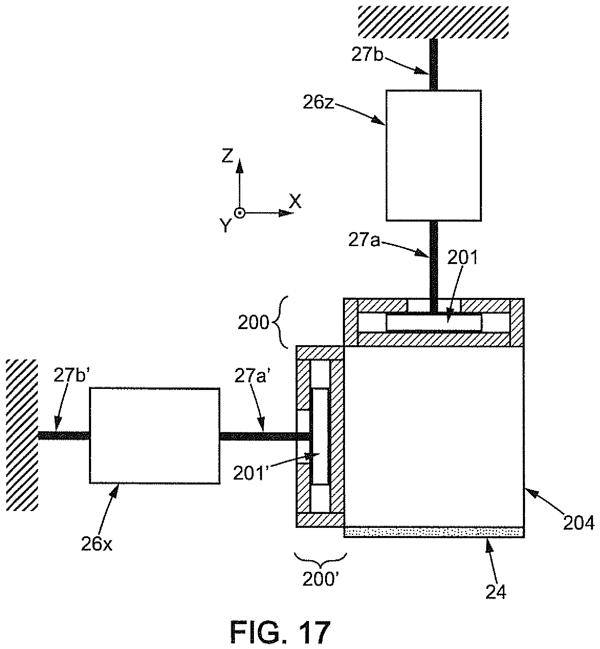

Afin d'entraîner l'organe d'entraînement 24 suivant non plus un mais deux axes, X et Z, on utilise simultanément deux actionneurs 26x et 26z (voir figure 17), placés suivant les directions : Z pour l'actionneur 26z et X pour l'actionneur 26x. L'axe d'entraînement de l'actionneur 26z est relié, par l'intermédiaire d'une tige 27a, à une pièce d'interface 201. L'axe d'entraînement de l'actionneur 26x est relié, par l'intermédiaire d'une tige 27a', à une pièce d'interface 201'. Les axes 27b et 27b' des actionneurs sont fixes par rapport à la base du système. La pièce d'interface 201 est maintenue par l'ensemble 200. La pièce d'interface 201' est maintenue par l'ensemble 200'. Une plaque 204, solidarise les ensembles 200 et 200' entre eux. L'organe d'entraînement 24 est fixé sur la plaque 204, sur la face opposée à la face de contact entre l'ensemble 200 et la plaque 204.

Dans ce cas, un mouvement de l'actionneur 26z, suivant son axe de travail Z, provoque un mouvement correspondant de la surface d'entraînement suivant le même axe Z. Dans le même temps, cela provoque un glissement de l'ensemble 200' par rapport à la pièce d'interface 201', cette pièce d'interface 201' restant immobile. De manière réciproque, un mouvement de l'actionneur 26x suivant son axe X provoquera un déplacement correspondant de l'organe d'entraînement 24 suivant l'axe X, la pièce d'interface 201 restant immobile. Enfin, un mouvement simultané des actionneurs 26z et 26x provoquera un déplacement combiné de l'organe d'entraînement suivant les directions X et Z.

Le principe d'un déplacement suivant deux axes décrit ci-dessus peut être extrapolé aux trois dimensions de l'espace en remplaçant la plaque 204 par un cube 204' et en plaçant trois actionneurs 26x, 26y et 26z maintenus par trois ensembles, ces trois ensembles étant fixés sur trois faces adjacentes d'un cube 204, et l'organe d'entraînement 24 étant placée sur l'une quelconque des trois autres faces. Les trois actionneurs étant reliés de façon solidaire à la base du système par des axes respectifs, un mouvement de leur axe 27 respectif selon chaque direction associée, est transmis jusqu'à la surface d'entraînement 24, qui reproduira ces mouvements, successifs ou simultanés. Ainsi, l'organe d'entraînement peut occuper n'importe quelle position (X,Y,Z) définie par les trois fenêtres dans l'espace et suivre n'importe quelle trajectoire, sa surface gardant toutefois une orientation constante. Son domaine de mouvement est défini à la fois par la course maximum des actionneurs 26x, 26y et 26z, par la dimension des ouvertures, ainsi que la dimension des pièces d'interface. Quelle que soit la position de l'organe médical souple allongé, un retrait d'urgence de celui-ci hors du module robotisé est donc toujours possible.

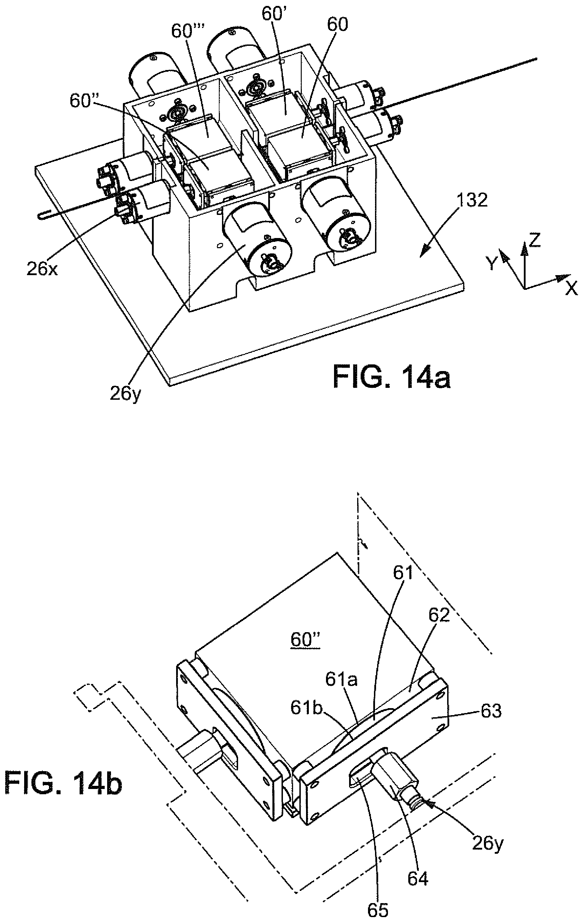

Une mise en œuvre pratique sera décrite plus loin en relation avec les figures 14a et 14b.

On a ainsi présenté un système d'actionnement d'un doigt d'entraînement selon 3 directions indépendantes de l'espace.

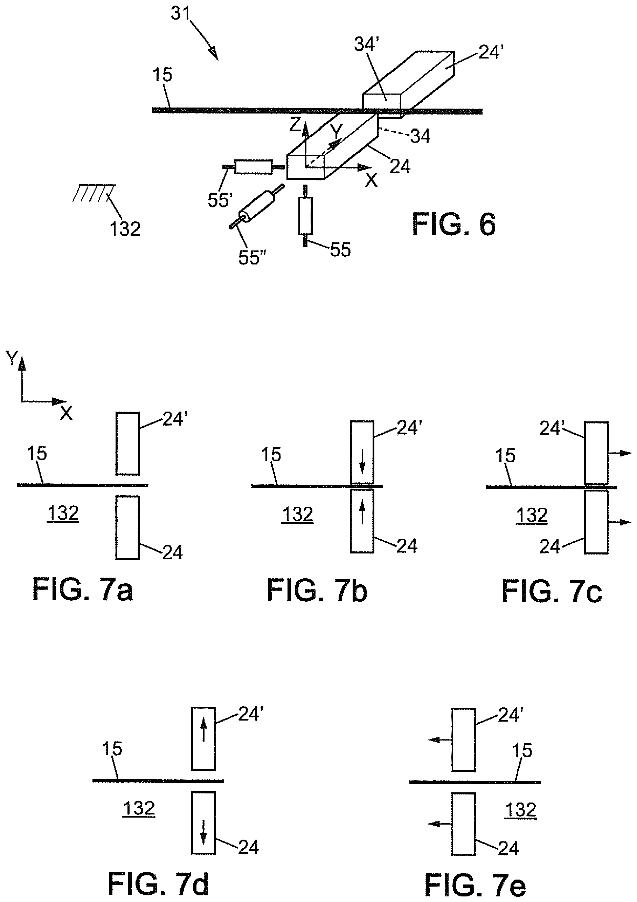

Sur la figure 6, on a représenté un module d'entraînement 131 selon un premier mode de réalisation. Ce module d'entraînement 131 est adapté pour entraîner un organe médical souple allongé 15 s'étendant selon une direction longitudinale X. On notera que la direction longitudinale X au niveau du module d'entraînement 131 n'est pas forcément la même que celle de l'organe médical souple allongé 15 au niveau de son extrémité mais qu'une translation et/ou une rotation de l'organe médical souple allongé 15 selon/autour de la direction longitudinale X au niveau du module d'entraînement 131 entraînera une translation et/ou une rotation de l'organe médical souple allongé 15, respectivement, selon/autour sa direction longitudinale au niveau de son extrémité.

Le module d'entraînement 131 comprend une base 132 et au moins un organe d'entraînement 24 monté mobile par rapport à la base 132. L'organe d'entraînement 24 est par exemple monté mobile par rapport à la base 132 comme expliqué ci-dessus en relation avec la figure 5.

Dans l'exemple présenté, le module d'entraînement 131 comprend en outre un deuxième organe d'entraînement 24'. L'organe d'entraînement 24, aussi appelé par la suite premier organe d'entraînement, et le deuxième organe d'entraînement 24' forment ensemble une paire d'organes d'entraînement 33. Une paire d'organes d'entraînement 33 comprend deux organes d'entraînement qui coopèrent ensemble pour générer un mouvement de l'organe médical souple allongé 15 par rapport à la base 132. Dans l'exemple présenté, le deuxième organe d'entraînement 24' est monté mobile par rapport à la base 132. Le deuxième organe d'entraînement 24' est par exemple monté mobile par rapport à la base 132 comme expliqué ci-dessus en relation avec la figure 5.

Le premier organe d'entraînement 24 et le deuxième organe d'entraînement 24' sont appariés pour des mouvements simultanés. Par exemple, les premier et deuxième organes d'entraînement 24, 24' peuvent être commandés individuellement indépendamment l'un de l'autre, mais selon des commandes respectives synchronisées. En variante, on peut prévoir une commande commune qui va être distribuée à l'un et à l'autre des premier et deuxième organes d'entraînement 24, 24' par une liaison mécanique ou électronique entre leurs systèmes de commande.

Chaque organe d'entraînement 24, 24' comporte une surface d'entraînement 34, 34' respectivement. L'organe médical souple allongé 15 est disposé entre les surfaces d'entraînement 34, 34' des organes d'entraînement 24, 24' d'une même paire. Pour fixer les idées, les surfaces d'entraînement 34, 34' sont espacées l'une de l'autre selon la direction Y.

La paire d'organes d'entraînement 24, 24' peut être placée dans une configuration libre, représentée sur la figure 6, dans laquelle la surface d'entraînement 34, 34' des organes d'entraînement 24, 24' de la paire d'organes d'entraînement 33 n'est pas en prise avec l'organe médical souple allongé 15.

La paire d'organes d'entraînement 33 est plaçable dans une configuration d'entraînement dans laquelle les surfaces d'entraînement 34, 34' des organes d'entraînement de la paire d'organes d'entraînement sont en prise avec l'organe médical souple allongé 15 à entraîner. La force appliquée par un organe d'entraînement sur l'organe médical souple allongé dans cette configuration est par exemple de l'ordre de quelques Newtons (5-30 N par exemple). Les moyens de rappel, décrits plus haut, sont par exemple agencés pour ramener la paire d'organes d'entraînement en configuration libre, ce qui permet de fournir une fonction sécuritaire, par exemple en cas de rupture d'alimentation électrique.

Pour placer la paire d'organes d'entraînement 33 alternativement dans les configurations libre et d'entraînement, on peut commander un déplacement relatif l'un vers l'autre des deux organes d'entraînement 24, 24'. Ce déplacement peut par exemple être le déplacement d'un organe d'entraînement 24 par rapport à la base, l'autre restant fixe. En variante, les deux organes d'entraînement 24, 24' peuvent tous deux se déplacer l'un vers l'autre par rapport à la base.

Dans l'exemple, on prévoit un déplacement selon la direction Y.

Dans le mode de réalisation présenté, les deux organes d'entraînement 24, 24' sont mobiles par rapport à la base selon un degré de liberté. Ce degré de liberté diffère de celui permettant le placement alternatif des organes d'entraînement entre les positions libre et d'entraînement. On prévoit notamment que les organes d'entraînement 24, 24' sont mobiles par rapport à la base selon un degré de liberté dans leur configuration d'entraînement. Ainsi, le déplacement des organes d'entraînement selon un degré de liberté dans leur configuration d'entraînement génère un déplacement de l'organe médical souple allongé par rapport à la base 132.

Un exemple sera décrit plus en détail ci-après en relation avec les figures 7a à 7e. Cet exemple décrit la génération d'un mouvement de translation de l'organe médical souple allongé selon sa direction longitudinale X.

La position de départ, représentée à la figure 7a, correspond à celle de la figure 6 décrite ci-dessus. Dans un premier temps, on passe de la configuration libre représentée à la figure 7a à la configuration d'entraînement (figure 7b). Selon l'exemple, ce passage se fait par un mouvement en sens opposé des deux organes d'entraînement selon la direction Y. L'amplitude de ce mouvement peut dépendre de l'organe médical souple allongé 15 à entraîner. Un guide, de diamètre inférieur au cathéter, pouvant nécessiter un mouvement de plus grande amplitude que le cathéter à partir d'une même position de départ.

En configuration d'entraînement, on génère un déplacement simultané dans le même sens des organes d'entraînement selon la direction longitudinale X selon un premier sens, ce qui génère un mouvement identique de l'organe médical souple allongé 15 (figure 7c).

On passe de la configuration d'entraînement représentée à la figure 7c à la configuration libre (figure 7d). Selon l'exemple, ce passage se fait par un mouvement en sens opposé des deux organes d'entraînement selon la direction Y, dans le sens opposé au sens pour faire passer les organes d'entraînement de la configuration d'entraînement à la configuration libre.

En configuration libre, on génère un déplacement simultané (ou non) dans le même sens des organes d'entraînement selon la direction longitudinale X selon un deuxième sens opposé au premier sens, ce qui ne génère pas de mouvement de l'organe médical souple allongé 15 (figure 7e). On est alors revenu à la configuration de départ.

On peut répéter les étapes ci-dessus de manière commandée cyclique pour générer une translation de l'organe médical souple allongé selon une course longue (par exemple de l'ordre de plusieurs mètres) selon la direction longitudinale X dans le premier sens.

Le déplacement de l'organe médical souple allongé selon une course longue selon la direction longitudinale X dans le deuxième sens peut se faire par une suite d'opérations opposées de celle qui vient d'être décrite.

La fréquence du cycle peut être réglable et commandable. En particulier, on peut prévoir une fréquence faible pour l'introduction de l'organe médical souple allongé dans le patient, voire plusieurs niveaux de fréquence faible, pour permettre en particulier une navigation lente dans les environnements difficiles. On peut prévoir une fréquence rapide, par exemple pour un retrait, voire un retrait d'urgence. Les amplitudes de déplacement pour chaque cycle peuvent également être réglables.

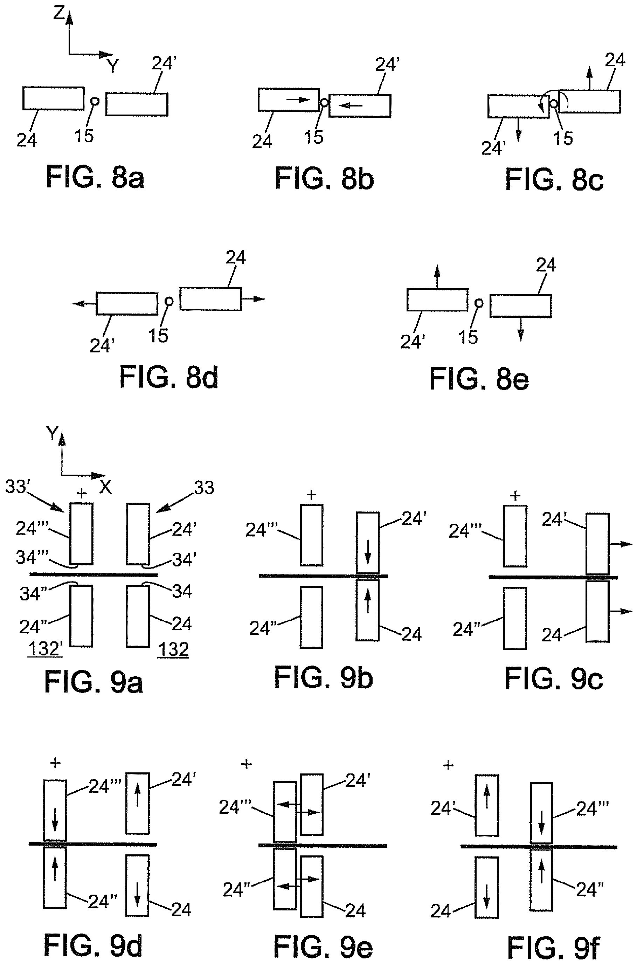

Pour la translation, des vitesses comprises entre 0,1 et 200 millimètres par secondes sont envisageables. L'invention sera décrit plus en détail ci-après en relation avec les figures 8a à 8e. Cet exemple décrit la génération d'un mouvement de rotation de l'organe médical souple allongé atour de sa direction longitudinale X.

La position de départ, représentée à la figure 8a, correspond à celle de la figure 6 décrite ci-dessus. Dans un premier temps, on passe de la configuration libre représentée à la figure 8a à la configuration d'entraînement (figure 8b). Selon l'exemple, ce passage se fait par un mouvement en sens opposé des deux organes d'entraînement selon la direction Y. Ce passage est le même que déjà décrit en relation avec les figures 7a, 7b ci-dessus.

En configuration d'entraînement, on génère un déplacement simultané en sens opposé des organes d'entraînement selon une direction Z transversale à la direction longitudinale X, différente de la direction Y, ce qui génère un mouvement de rotation de l'organe médical souple allongé 15 (figure 8c) autour de la direction longitudinale X. En particulier, l'organe médical souple allongé roule, de préférence sans glissement, sur les surfaces d'entraînement 34, 34' des organes d'entraînement 24, 24'. En variante, on pourrait déplacer un seul des deux organes d'entraînement, l'autre restant fixe.

On passe de la configuration d'entraînement représentée à la figure 8c à la configuration libre (figure 8d). Selon l'exemple, ce passage se fait par un mouvement en sens opposé des deux organes d'entraînement selon la direction Y, dans le sens opposé au sens pour faire passer les organes d'entraînement de la configuration d'entraînement à la configuration libre.

En configuration libre, on génère un déplacement simultané (ou non) des organes d'entraînement selon la direction Z, opposé au déplacement décrit ci-dessus en relation avec la figure 8c, ce qui ne génère pas de mouvement de l'organe médical souple allongé 15 (figure 8e). On est alors revenu à la configuration de départ.

On peut répéter les étapes ci-dessus de manière commandée cyclique pour générer une rotation de l'organe médical souple allongé selon une course longue (par exemple de plusieurs fois 360°) autour de la direction longitudinale X dans un premier sens de rotation.

Le déplacement de l'organe médical souple allongé selon une course longue autour de la direction longitudinale X dans le deuxième sens de rotation opposé au premier peut se faire par une suite d'opérations opposées de celle qui vient d'être décrite.

Dans la description ci-dessus, le degré de rotation de l'extrémité libre de l'organe médical souple à l'intérieur du corps du patient peut être surveillé par imagerie. Toutefois, on peut également, en variante ou en complément, chercher à contrôler en amont l'amplitude de la rotation appliquée à l'organe médical souple au niveau du module d'entraînement. Ceci passe par une connaissance du diamètre de l'organe médical souple allongé au niveau des organes d'entraînement 24, 24'. En effet, l'angle de rotation de l'organe médical souple allongé pour un déplacement donné des organes d'actionnement dépend du rapport entre le diamètre de l'organe médical souple allongé et la course des organes d'entraînement. Ce diamètre peut être prédéfini et stocké dans le poste de commande 11. Il suffit d'informer au préalable le poste de commande 11 du type de cathéter utilisé, le type en question comprenant le diamètre. On peut également, en variante, détecter in situ le diamètre de l'organe médical souple allongé. Si la configuration libre de chaque organe d'entraînement constitue une position de référence, on peut connaître la position de l'organe d'entraînement en configuration d'entraînement par exemple en utilisant un système de codage sur l'actionneur associé à chaque organe d'entraînement et permettant de déplacer l'organe d'entraînement de sa configuration libre à sa configuration d'entraînement.

En connaissant la position des deux organes d'entraînement en configuration d'entraînement, et en connaissant l'écart entre les surfaces d'entraînement 34, 34' des deux organes d'entraînement dans leur configuration libre, on peut déterminer l'écart entre les deux surfaces d'entraînement en configuration d'entraînement et, partant, le diamètre de l'organe médical souple allongé.

Cette connaissance peut également être utilisée pour détecter la fin d'un mouvement de retrait de l'organe médical souple allongé. En effet, si le poste de commande 11 détecte une variation soudaine du diamètre détecté au cours du temps lors d'une commande de retrait de l'organe médical souple allongé, cela signifie vraisemblablement que l'organe médical souple allongé a été intégralement retiré du patient, et même du module. Le diamètre détecté alors peut être soit nul, soit par exemple le diamètre du guide si celui-ci s'étend alors entre les deux organes d'entraînement.

On peut également maîtriser le serrage de l'organe médical souple allongé en configuration d'entraînement.

En effet, en configuration d'entraînement, le courant appliqué aux actionneurs est proportionnel à la force de serrage appliquée sur l'organe médical souple allongé. La connaissance de ce courant permet donc de déterminer le serrage appliqué au cathéter. En pratique, on pourra prévoir au niveau du poste de commande 11 différentes consignes en courant pour les actionneurs, disposés dans une plage de serrage acceptable en dehors de laquelle on risquerait soit de voir glisser l'organe médical souple allongé hors de prise, soit de détériorer l'organe médical souple allongé par une trop importante sollicitation mécanique par les organes d'entraînement.

La maitrise du serrage de l'organe médical souple allongé peut être faite pour n'importe quel mouvement appliqué au cathéter, pas seulement pour le mouvement de rotation décrit ci-dessus.

La détermination du diamètre de l'organe médical souple allongé pourrait être faite pour d'autres mises en œuvre de déplacement de cathéter que les commandes répétées cycliques décrites ici.

Ainsi, indépendamment des commandes répétées cycliques décrites ici, il semble 'un autre example de réalisation non-revendiqué se rapporte à un module robotisé d'entraînement d'organe médical souple allongé comprenant :
- une base 132,
- une paire 33 d'organes d'entraînement 24, 24' présentant chacun une surface d'entraînement 34, 34', la paire 33 d'organes d'entraînement 24, 24' étant plaçable par au moins un actionneur 26 dans une configuration d'entraînement dans laquelle les surfaces d'entraînement 34, 34' des organes d'entraînement 24, 24' de la paire 33 d'organes d'entraînement 24, 24' sont en prise avec l'organe médical souple allongé à entraîner et disposés de part et d'autre de celui-ci,
la paire 33 d'organes d'entraînement 24, 24' étant montée mobile par rapport à la base 132 selon un degré de liberté entre une première et une deuxième positions,
- un organe de commande 18, 11 adapté pour commander à partir d'un signal représentatif relatif à l'actionneur 26 (par exemple de manière répétée cyclique) un déplacement par rapport à la base 132 des organes d'entraînement 24, 24' de la paire 33 d'organes d'entraînement 24, 24' en configuration d'entraînement de la première à la deuxième position, entraînant ainsi l'organe médical souple allongé par rapport à la base 132.

En particulier, le signal représentatif relatif à l'actionneur permet de déterminer un écartement entre les surfaces d'entrainement 34, 34', l'organe de commande 18, 11 commandant un déplacement déterminé à partir de l'écartement par rapport à la base 132 des organes d'entraînement 24, 24' de la paire 33 d'organes d'entraînement 24, 24' entrainant ainsi une rotation d'amplitude contrôlée l'organe médical souple allongé par rapport à la base 132.

En particulier, le signal représentatif relatif à l'actionneur permet de maitriser une force de serrage appliquée à l'organe médical souple allongé dans une gamme admissible de forces de serrage.

Dans les deux exemples de réalisation ci-dessus, on a décrit un déplacement séquencé au cours duquel on attend d'avoir terminé le déplacement d'un organe d'entraînement selon une direction pour entamer un autre déplacement.

Toutefois, étant donné que les actionnements des organes d'entraînement selon divers degrés de liberté peuvent être rendus indépendants en utilisant de manière indépendante les trois systèmes d'actionnement 55, 55', 55" décrits plus haut, on pourrait mettre en œuvre de manière simultanée le déplacement d'un organe d'entraînement selon deux degrés de liberté. Par exemple, le déplacement des organes d'entraînement de la position de la figure 8c à celle de la figure 8e pourrait inclure une phase intermédiaire entre une première phase de pur écartement et une deuxième phase de pur retour à la position initiale, où ces deux mouvements sont combinés. Une phase intermédiaire similaire est également envisageable entre la position de la figure 8d et la position de la figure 8b entre la phase de pur retour à la position initiale et une phase de pur rapprochement. En tirant le trait, on pourrait n'avoir plus de phases de pur retour à la position initiale, pur écartement et pur rapprochement, dans la mesure où on ne risque pas de générer de mouvements parasites de l'organe médical souple allongé.

Par ailleurs, alors qu'on a présenté de manière indépendante aux figures 7a-7e un mouvement de translation pure de l'organe médical souple allongé, et aux figures 8a-8e un mouvement de rotation pure, ces deux mouvements pourraient alternativement être combinés. Il suffirait, en configuration en prise, de combiner les mouvements adaptés des organes d'entraînement pour générer simultanément translation et rotation.

L'exemple précédent comprend une unique paire d'organes d'entraînement.

En variante, on pourrait prévoir plusieurs paires d'organes d'entraînement. Par exemple, à titre descriptif, on pourrait prévoir deux paires d'organes d'entraînement. Les organes d'entraînement 24", 24‴ de la deuxième paire 33' peuvent être similaires à ceux de la première paire, et en particulier comporter des surfaces d'entraînement 34", 34‴, et être actionnés depuis le poste de commande 11 distant, voire le boîtier de commande 18 local selon des mises en œuvre similaires à celles de la première paire. La première paire 33 et la deuxième paire 33' d'organes d'entraînement peuvent être décalées l'une par rapport à l'autre selon l'axe longitudinal X de l'organe médial souple allongé. Selon un premier exemple, les deux paires 33, 33' peuvent être prévues coplanaires dans leur configuration libre. C'est-à-dire qu'elles peuvent être prévues vis-à-vis d'une base 132 commune aux deux paires. En variante, les bases 132, 132' de chaque paire pourraient être indépendantes, voire non coplanaires.

Les actionnements des deux paires peuvent être synchronisés. Par exemple, les actionnement des deux paires peuvent générer des mouvements identiques simultanés des deux paires.

En variante, les deux paires peuvent être actionnés de manière synchronisées pour générer des mouvements décalés en phase. C'est-à-dire qu'une première paire 33 peut être en configuration d'entraînement pendant qu'une autre paire est en configuration libre, et vice-versa. Par exemple, il y a toujours au moins une paire en configuration d'entraînement. A chaque moment donné, il peut s'agir de la première, de la deuxième, voire des deux en même temps. Une telle configuration permet d'améliorer le maintien de l'organe médical souple allongé. Notamment quand l'organe médical souple allongé est déplacé en frottant contre une zone anatomique du patient, il faut pouvoir assurer un maintien suffisant de celui-ci pour vaincre la résistance locale au déplacement. Ceci est rendu d'autant plus difficile quand l'organe médical souple allongé est glissant, par exemple du fait de son maintien dans une solution.

Un exemple est donné à titre illustratif sur les figures 9a à 9f pour un mode d'entraînement en translation. Sur ces figures, une référence fixe au cours du temps est désignée par le signe « + ». Le mouvement de la première paire, représenté aux figures 9a à 9e a déjà été décrit ci-dessus en relation avec les figures 7a à 7e. La figure 9f représente la même position que la figure 9b, le déplacement étant cyclique.

Les figures 9b à 9f représentent les déplacements de la deuxième paire 33' au cours d'un cycle. Ces déplacements sont déphasés par rapport à ceux de la première paire, la position illustrée sur la figure 9d pour la deuxième paire correspondant à celle de la figure 9b pour la première paire, et ainsi de suite.

Les deux paires sont espacées de manière à éviter toute collision, notamment tel que représenté à la figure 9e, où la deuxième paire est soumise à un déplacement dans le sens de l'avancement de l'organe médical souple allongé et où la première paire est soumise à un déplacement en sens opposé.

A titre illustratif, la figure 9a peut représenter un état initial dans lequel les deux paires se situent à distance de l'organe médical souple allongé. Lors de la mise en route du système, la première paire sera commandée puis, de manière déphasée, la deuxième paire.

Cette mise en œuvre s'applique pour d'autres mouvements que la translation. Cette mise en œuvre s'applique pour plus de deux paires. Dans ce cas, les paires sont, le cas échéants, toutes déphasées les unes par rapport aux autres, ou certaines paires peuvent être en phase les unes avec les autres.

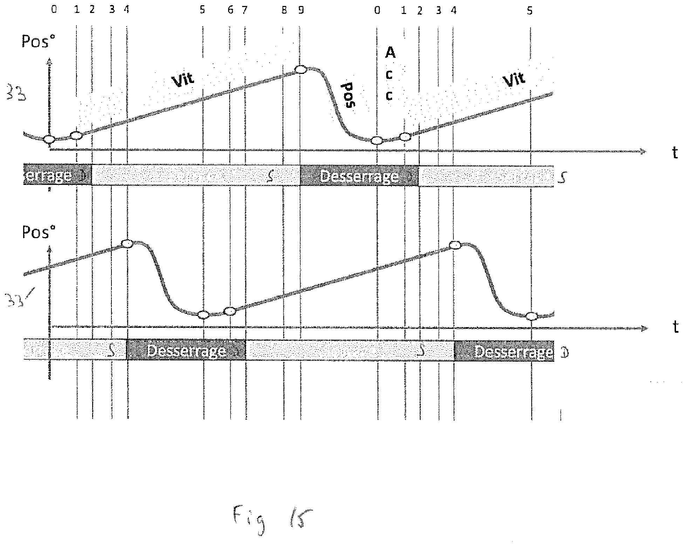

La figure 15 décrit un exemple concret de synchronisation des deux paires 33, 33'. Le cycle ci-dessous est décrit par référence à la première paire 33, sachant que la paire 33' est en opposition de phase par rapport à celle-ci.
- Etape 0 : accélération pour atteindre la vitesse cible
- Etape 1 : la vitesse étant atteinte, on la maintient à un niveau constant et on lance l'ordre de serrage, avec l'objectif d'atteindre le serrage effectif au début de l'étape 2 ; pendant ce temps, la deuxième paire 33' est encore à pleine vitesse, avec serrage activé
- Etape 2 : on passe en serrage et reste à vitesse constante
- Etape 3 : on reste en serrage et reste à vitesse constante ; à ce moment, la deuxième paire 33' reçoit l'ordre de desserrage, qui sera effectif au début de l'étape 4 après un certain délai (lié au temps de réponse mécanique et électronique de l'ensemble du système) ; au total, on peut considérer que l'on aura eu une période d'entraînement simultané par les deux paires commençant au début de l'étape 2 et se terminant au début de l'étape 4.
- Etape 3 : on reste en serrage et vitesse constante ;
- Etape 4 : on reste en serrage et vitesse constante ; pendant ce temps, la deuxième paire 33' retourne à sa position d'origine et attend, immobile, à sa position d'origine ; ce temps d'attente est variable en fonction de la vitesse de consigne de translation et de rotation, et également du temps total de cycle.
- Etape 5 : on reste en serrage et vitesse constante ; la deuxième paire 33' arrive au début de son cycle, c'est-à-dire l'équivalent de l'étape 0 pour la première paire 33.
- Etape 6 : on reste en serrage et vitesse constante ; la deuxième paire 33' termine son accélération et arrive en début d'étape à vitesse constante (équivalent de l'étape 1 pour la première paire 33)
- Etape 7 : on reste en serrage et vitesse constante ; pour la deuxième paire 33', le serrage devient effectif
- Etape 8 : on envoie l'ordre de desserrage, tout en maintenant la vitesse constante ; en effet, l'ordre de desserrage va nécessairement mettre un certain délai à être effectif, et il est donc nécessaire de continuer à vitesse constante pendant ce délai ;
- Etape 9 : on considère que le desserrage est maintenant effectif, et on envoie l'ordre de retour à la position initiale, en vue du cycle suivant, puis le retour étant effectif, on attend à la position initiale jusqu'au début du cycle suivant.

Dans le cas où on détecte le diamètre de l'organe médical souple allongé avec au moins deux paires d'organes d'entraînement, on peut détecter qu'on atteint la fin d'une étape de retrait de l'organe médical souple allongé si deux paires d'organes d'entraînement permettent de déterminer des diamètres différents. Cela surviendra quand une paire amont détecte encore la présence de l'organe médical souple allongé entre ses organes d'entraînement et qu'une paire aval ne la détecte plus (ne détecte plus que soit un guide, soit rien). Une telle détection permet d'arrêter de commander les organes d'entraînement aval s'il n'est pas prévu que ceux-ci entraînent le guide. Par ailleurs, indépendamment, une telle détection permet d'arrêter, au besoin, un retrait complet de l'organe médical souple allongé, ce qui permet le cas échéant de procéder à une nouvelle insertion de l'organe médical souple allongé dans le patient sans intervention manuelle pour ré-engager l'organe médical souple allongé dans le module d'entraînement.

Dans les modes de réalisation décrits ci-dessus, les organes d'entraînement sont disposés de manière symétrique par rapport à un plan général médian de l'organe médical souple allongé.

Toutefois, en variante, les organes d'entraînement peuvent être montés mobiles par rapport à la base 132 pour décaler latéralement l'organe médical souple allongé localement par rapport à son axe longitudinal neutre X'. L'axe longitudinal neutre X' est défini par l'axe longitudinal naturellement occupé par l'organe médical souple allongé sans aucune sollicitation par des organes d'entraînement 24. Un tel décalage latéral est possible en générant un mouvement simultané des organes d'entraînement 24, 24' en configuration en prise dans le même sens selon une direction transversale (axe Y ou Z, ou une combinaison selon ces deux axes) par rapport à la configuration en prise au niveau de l'axe longitudinal neutre.

Le cas échéant, si on utilise plusieurs paires d'organes d'entraînement, celles-ci peuvent être disposées, en configuration en prise, selon des décalages latéraux différents par rapport à l'axe longitudinal neutre. On peut alors mettre en œuvre un actionnement de type « manivelle ».

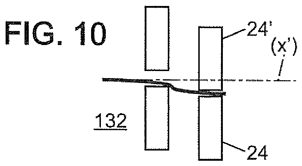

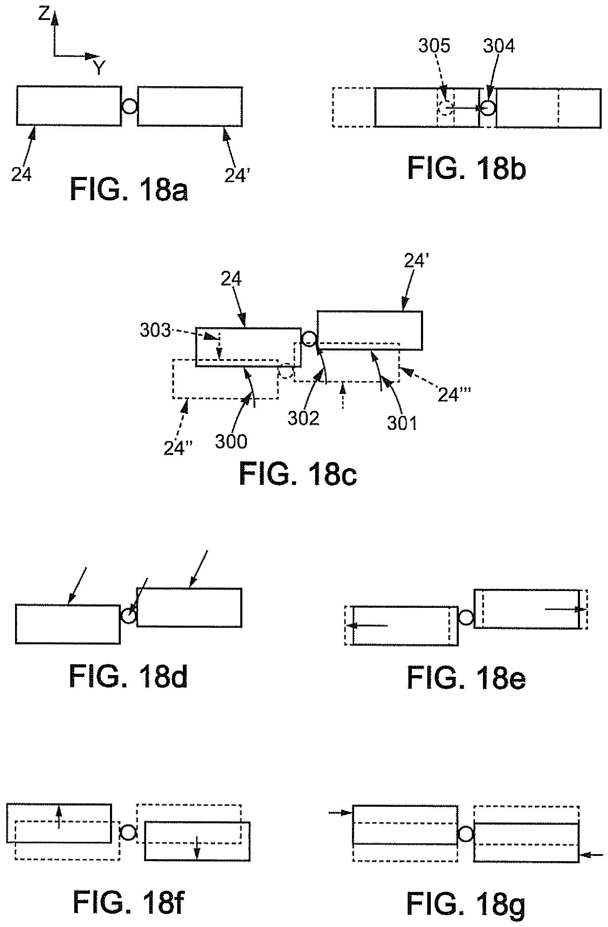

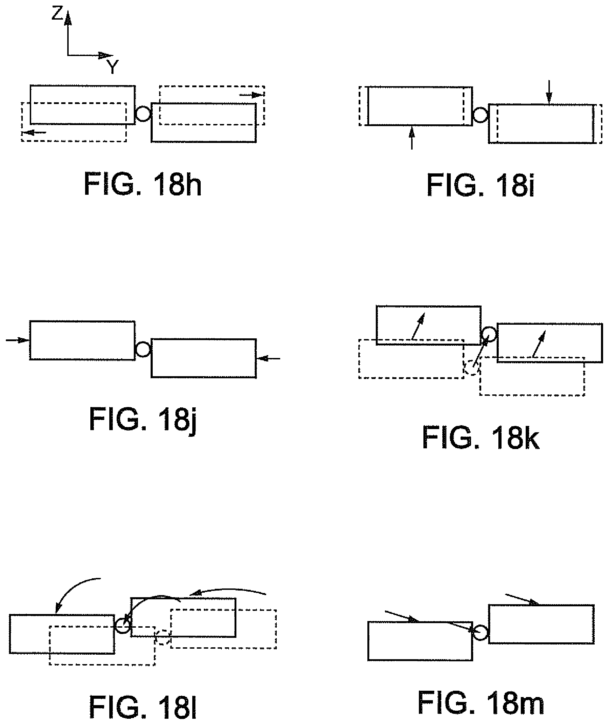

La figure 18a est une vue de face correspondant à la configuration de la figure 10, prise dans le plan de la première paire d'organes d'entraînement 24, 24' représentés sur la figure 10. L'organe médical souple allongé 15, dans ce plan, est représenté en traits pleins. La position de l'organe médical souple allongé dans le plan de la deuxième paire d'organes d'entraînement est représentée en pointillés. Le même code traits pleins/traits pointillés est appliqué à la deuxième paire d'organes d'entraînement est en configuration d'entraînement pour la suite de la description (figures 18a à 18m).

Les figures 18a à 18m décrivent une mise en œuvre pour ce mode. Comme sur la figure 10, les deux paires d'organes d'entraînement sont représentées l'une derrière l'autre. La paire (24", 24‴) située derrière est représentée en pointillés. L'organe médical souple allongé est représenté deux fois, sous la forme de deux coupes, l'une 304 au niveau de la première paire (au premier plan), en trait plein, et l'autre au niveau de la deuxième paire d'organes d'entraînement (à l'arrière-plan), en pointillés :
- 18a : position initiale.
- 18b : décalage à droite de la première paire
- 18c : le barycentre de la première paire (24, 24') décrit un arc de cercle autour de l'axe défini par l'organe médical souple allongé à la figure 18a. Simultanément, les deux paires ont un mouvement de translation suivant Z, chaque organe d'entraînement ayant un sens opposé à l'autre organe d'entraînement de la même paire. Pour chaque élément de la première paire (24, 24'), on voit donc que la trajectoire est la somme vectorielle d'une trajectoire rectiligne et d'une trajectoire circulaire. En résultante, on obtient donc deux courbes montantes, avec toutefois un rayon de courbure plus faible pour la courbe 300 de l'organe d'entraînement 24 que la courbe 301 de l'organe d'entraînement 24'. Cela est lié au fait que la trajectoire de l'organe d'entraînement 24 est la somme d'un arc de cercle identique à la trajectoire 302 de l'organe médical souple allongé, dirigée approximativement vers le haut, et d'une trajectoire rectiligne dirigée vers le bas, identique à la trajectoire 303 de l'actionneur 24", et dirigée vers le bas,
- 18d : retour de la première paire à la position d'origine, de sorte que l'organe médical souple allongé est à nouveau guidé suivant une ligne droite,
- 18e : desserrage de la première paire, la deuxième paire restant serrée,
- 18f : mouvement suivant Z des deux organes d'entraînement de la première paire, en sens opposé, afin d'obtenir une position finale en Z symétrique de la position initiale par rapport à l'organe médical souple allongé,
- 18g : serrage de la première paire,
- 18h : desserrage de la deuxième paire, la première paire restant serrée,
- 18i : mouvement de la deuxième paire suivant Z identique à 18f pour la première paire,
- 18j : serrage de la deuxième paire,
- 18k : retour de la première paire d'actionneur à la position finale de 18c,
- 18l : mouvement similaire 18c mais sur un arc de cercle différent [à noter qu'il serait possible d'utiliser toujours le même arc de cercle, plutôt que d'utiliser une succession d'arcs de cercles qui formeront un cercle complet, comme présenté ici].
- 18m : retour de la première paire à la position, comme en 18d,
- Etc...

La mise en œuvre qui vient d'être décrite représente schématiquement un exemple non limitatif de mise en œuvre combinée de déplacements de deux organes d'entraînement d'une même paire selon deux degrés de liberté combinés, de mise en œuvre successive de déplacements de deux organes d'entraînement d'une même paire selon deux degrés de liberté différents, de combinaison de la mise en œuvre de deux paires indépendantes d'organes d'entraînement.

Un exemple de mise en œuvre pratique d'un tel système est présenté ci-après en relation avec les figures 14a et 14b. Cet exemple de réalisation est fourni de manière illustrative uniquement d'un exemple de réalisation concret d'un système d'actionnement.

La figure 14a comprend une base 132 fixe commune à quatre systèmes d'actionnement. Chaque système d'actionnement commande le déplacement d'un organe d'entraînement respectif, non représenté, mais solidaire d'un cube respectif 60, 60', 60", 60‴. Les cubes 60, 60', 60", 60‴ correspondent respectivement aux organes d'entraînement 24, 24', 24", 24‴ sur la figure 9a, sensiblement selon la même orientation.

Par la suite, seule l'opération d'un cube sera décrite. On fait par exemple référence au cube 60". Le cube 60" est associé à trois actionneurs 26x, 26y, 26z (ce dernier non visible, similaire en tout point aux actionneurs 26x et 26y, et situé sous la base 132. L'actionneur 26y est utilisé pour déplacer le cube 60" selon la direction Y, tout en autorisant un déplacement du cube 60" à la fois selon les directions X et Z par rapport à l'actionneur 26y dans une certaine plage de déplacement.

Comme visible sur la figure 14b, l'actionneur 26y présente une extrémité solidaire d'un disque 61 de large diamètre. Ce disque 61 est placé dans une fente 62 ménagée entre le cube 60" et une plaque 63 solidaire de celui-ci. En particulier, l'épaisseur de la fente 62 et du disque 61 se correspondent, de manière à ce qu'une surface 61a du disque soit en contact du cube 60" et qu'une surface opposée 61b soit en contact de la plaque 63.

Le bras 64 passe à travers une fenêtre 65 ménagée dans la plaque 63. La fenêtre 65 est de forme telle que le disque 61 ne peut pas sortir de la fente 62 à travers la fenêtre 65. La fenêtre 65 définit la plage de déplacement autorisée du cube par rapport à l'actionneur 26y selon les directions Y et Z.

Les autres actionneurs présentent une configuration similaire selon leurs orientations respectives.

Par conséquent, lors de l'extension de l'actionneur 26y, le disque 61 pousse sur le cube 60" selon la direction Y, et génère un déplacement de celui-ci selon cette direction. Lors de la rétraction de l'actionneur 26y, le disque 61 tire sur la plaque 63 selon la direction Y, et génère un déplacement du cube 60" solidaire de celle-ci selon cette direction. Ces déplacements sont autorisés dans la plage de déplacement autorisé par les fenêtres des plaques associées aux actionneurs 26x et 26z.

Lorsqu'un autre actionneur, par exemple l'actionneur 26x, génère un déplacement du cube 60" selon la direction X de la même manière, ce déplacement est possible dans la limite autorisée par la dimension de la fenêtre 65 selon la direction X (et idem pour la plaque associée à l'actionneur 26z dans cet exemple).

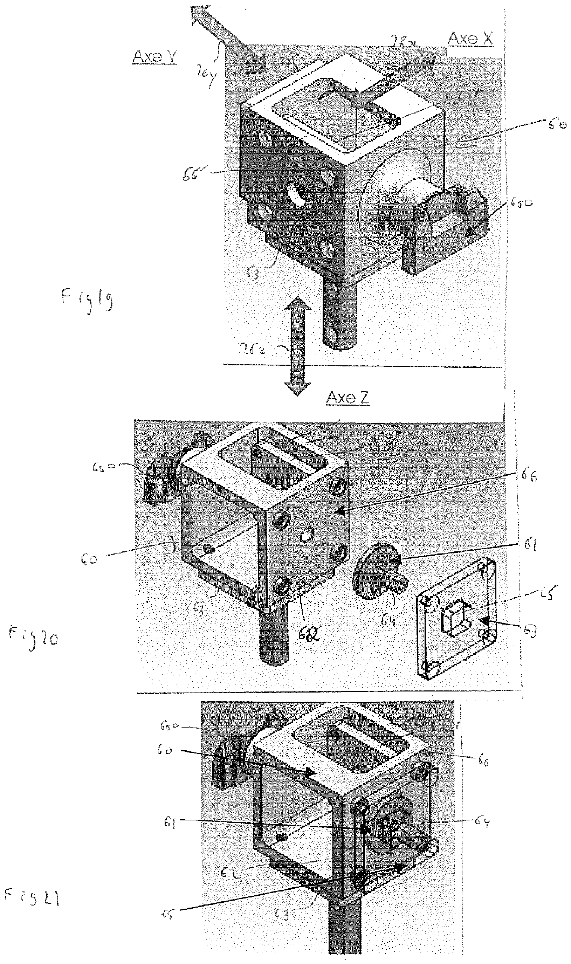

La figure 19 est une vue en perspective d'un autre exemple de réalisation d'un cube transmettant le mouvement des trois actionneurs à l'organe d'entraînement.

Le cube 60 comprend toujours les trois mêmes actionneurs 26x, 26y et 26z, respectivement selon les directions X, Y et Z. L'action de ces actionneurs 26x, 26y et 26z est matérialisée par une double flèche. Deux des interfaces entre actionneurs 26y et 26z et cube 60 sont disposées sur les faces extérieures du cube 60 comme dans le mode de réalisation précédent. L'une des interfaces, entre actionneur 26x et cube 60, fonctionne selon un principe similaire mais est au contraire disposée à l'intérieur du cube 60, ce qui permet un gain de compacité de l'ensemble constitué par ces interfaces et le cube 60. Cette interface comprend une plaque 63' disposée donc à l'intérieur du cube 60, en regard d'une paroi intérieure 66' du cube 60. Sur l'une des faces du cube 60 ne comprenant pas d'interface d'actionneur, est fixé un embout 650 de touche. Cet embout 650 va pouvoir porter de manière solidaire et fixe l'organe d'entraînement 24 présenté au niveau de la figure 6.

La figure 20 est une autre vue en perspective d'un autre exemple de réalisation d'un cube transmettant le mouvement des trois actionneurs à l'organe d'entraînement.

De manière plus détaillée, pour l'une des interfaces extérieures d'actionneur, celle de l'actionneur 26y, on retrouve le disque 60 et le bras 64 formant le patin, ce bras 64 passant au travers de la fenêtre 65 de la plaque 63. Le disque 61 se déplace de manière semblable au mode de réalisation précédent dans la fente 62. L'ensemble est montré en vue éclatée.

L'interface intérieure pour l'actionneur 26x avec sa plaque 63' fonctionne de manière similaire, mais le déplacement de son disque 61 dans le plan de la plaque 63' se fait entre la paroi intérieure 66' du cube 60 et cette plaque 63' dans une fente 62'. Par exemple, comme précédemment, la poussée du disque 61, perpendiculairement à la surface de la paroi intérieure 66' du cube 60 et/ou de la plaque 63', permet le déplacement du cube 60 selon l'axe X uniquement.

La figure 21 est encore une autre vue en perspective d'un autre exemple de réalisation d'un cube transmettant le mouvement des trois actionneurs à l'organe d'entraînement. Cette figure est similaire à la figure précédente, mais cette fois-ci, l'ensemble est montré de façon montée, et non plus en vue éclatée.

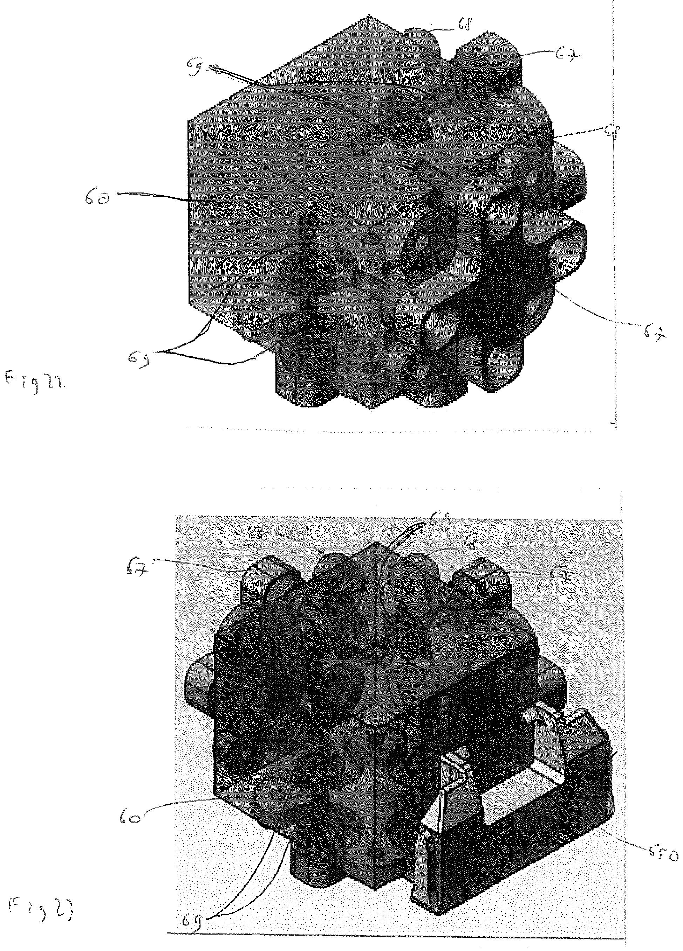

La figure 22 est une vue en perspective d'encore un autre exemple de réalisation d'un cube transmettant le mouvement des trois actionneurs à l'organe d'entraînement.

Le fonctionnement du cube 60 est semblable à celui du mode de réalisation précédent, mais sa structure est différente. En effet, le disque 61 a été remplacé par une croix 67. Cette forme en croix 67 du patin de pression permet de mieux répartir les efforts au niveau du cube 60 et de limiter les arcboutements. Des pions 69 à collerette prolongent la croix 67 au niveau de l'extrémité de ses 4 branches vers l'intérieur du cube 60. Une autre croix 68 dont les branches sont disposées en quinconce à celles de la croix 67 est située entre la paroi extérieure du cube 60 et la croix 67. L'actionneur pousse par exemple au niveau du centre de la croix 67 qui est en retour pousse le cube 60 au niveau des 4 pions 69 à collerette lesquels répartissent donc l'effort de poussée sur l'ensemble de la face du cube 60.

La figure 23 est une autre vue en perspective d'encore un autre exemple de réalisation d'un cube transmettant le mouvement des trois actionneurs à l'organe d'entraînement. Le cube 60 est vu d'un autre côté. Le cube 60 porte en plus un embout 650 de touche également solidaire et fixe de l'organe d'entraînement 24 présenté au niveau de la figure 6. Dans ce mode de réalisation également, selon une variante, l'une des interfaces d'actionneurs peut être disposée à l'intérieur du cube 60, comme dans le mode de réalisation précédent.

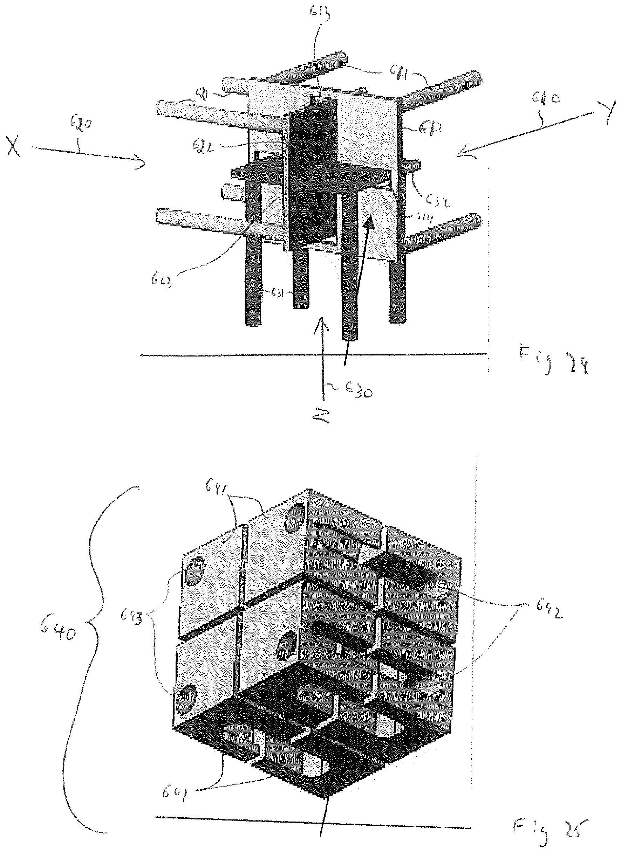

La figure 24 est une vue en perspective d'un exemple de réalisation de l'intersection et de l'imbrication des interfaces entre actionneurs d'une part et socle d'organe d'entraînement d'autre part.

Trois actionneurs 610, 620, 630 exercent un effort selon trois directions Y, X, Z, orthogonales entre elles.

L'actionneur 610 exerce son effort selon la direction Y par l'intermédiaire de 4 barres 611 poussant aux 4 coins d'une première plaque 612 de pression qui constitue l'interface entre l'actionneur 610 et le socle d'organe d'entraînement. La première plaque 612 comprend une première ouverture 613 traversée par une deuxième plaque 622 constituant l'interface entre l'actionneur 620 et le socle d'organe d'entraînement. Cette première ouverture 613 comprend un débattement dans la direction X de manière à permettre la course de l'actionneur 620 et de la deuxième plaque 622 associée selon la direction X sans déplacer la première plaque 612. La première plaque 612 comprend une deuxième ouverture 614 traversée par une troisième plaque 632 constituant l'interface entre l'actionneur 630 et le socle d'organe d'entraînement. Cette deuxième ouverture 614 comprend un débattement dans la direction Z de manière à permettre la course de l'actionneur 630 et de la troisième plaque 632 associée selon la direction Z sans déplacer la première plaque 612.

L'actionneur 620 exerce son effort selon la direction X par l'intermédiaire de 4 barres 621 poussant aux 4 coins d'une deuxième plaque 622 de pression qui constitue l'interface entre l'actionneur 620 et le socle d'organe d'entraînement. La deuxième plaque 622 comprend une troisième ouverture 623 traversée par une troisième plaque 632 constituant l'interface entre l'actionneur 630 et le socle d'organe d'entraînement. Cette troisième ouverture 623 comprend un débattement dans la direction Z de manière à permettre la course de l'actionneur 630 et de la troisième plaque 632 associée selon la direction Z sans déplacer la deuxième plaque 622.

L'actionneur 630 exerce son effort selon la direction Z par l'intermédiaire de 4 barres 631 poussant aux 4 coins d'une deuxième plaque 632 de pression qui constitue l'interface entre l'actionneur 630 et le socle d'organe d'entraînement. La troisième plaque 632 ne comprend aucune ouverture.

La figure 25 est une vue en perspective d'un exemple de réalisation du socle d'organe d'entraînement.

Le socle de l'organe d'entraînement comprend un cube 640 qui pourrait être l'un quelconque des cubes 60, 60', 60" ou 60‴ de la figure 14a. Ce cube 640 comprend 8 petits cubes 641 assemblés ensemble aux 8 sommets du cube 640. En réalité, les petits cubes 641 ne sont que des portions de petits cubes sur 3 faces. Sur certaines faces des petits cubes 641, sont disposées des ouvertures circulaires 643 tandis que sur d'autres faces des petits cubes 641 sont disposées des ouvertures oblongues 642. Les ouvertures circulaires 643, comme les ouvertures oblongues 642, sont destinées à recevoir les barres 611, 621 et 631 des différentes plaques 612, 622 et 632.

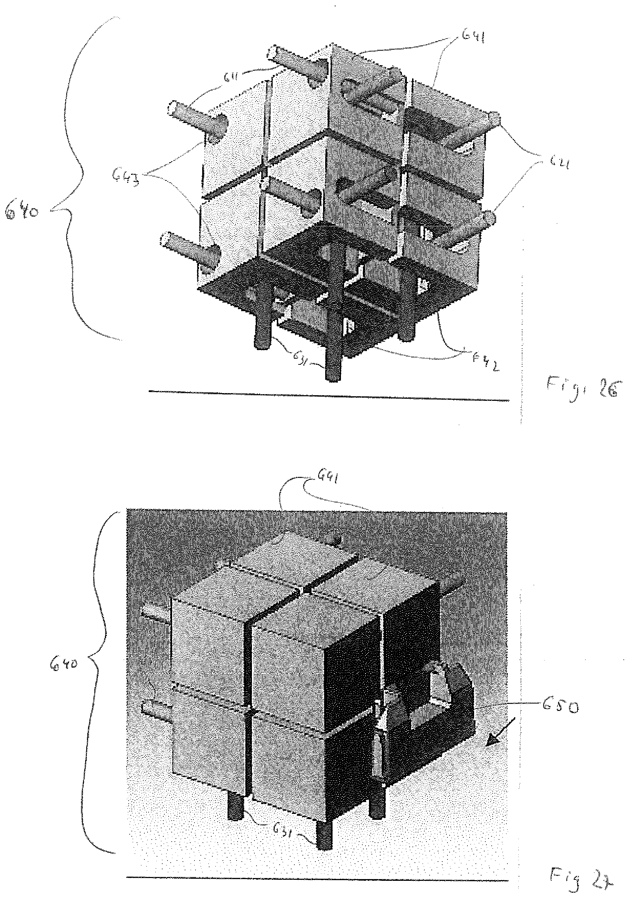

La figure 26 est une vue en perspective d'un exemple de réalisation d'assemblage entre d'une part les interfaces et d'autre part le socle d'organe d'entraînement.

Les barres 611, 621 et 631 des différentes plaques 612, 622 et 632 peuvent s'enfoncer plus ou moins dans les différentes ouvertures 642 et 643, permettant ainsi respectivement aux plaques 612, 622 et 632 de déplacer le cube 640 respectivement dans les directions Y, X et Z. Les plaques 612, 622 et 632 poussent ou tirent les petits cubes 641 formant ensemble le cube 640.

La figure 27 est une autre vue en perspective d'un exemple de réalisation d'assemblage entre d'une part les interfaces et d'autre part le socle d'organe d'entraînement.

De ce côté, les petits cubes 641 ne comportent aucune ouverture, mais seulement un embout 650 de touche. Cet embout 650 va pouvoir porter de manière solidaire et fixe l'organe d'entraînement 24 présenté au niveau de la figure 6.

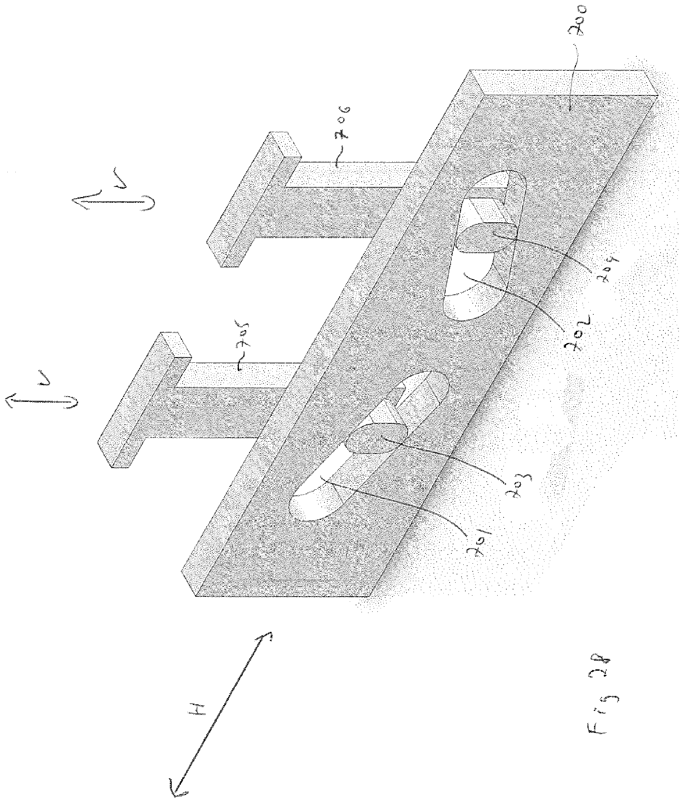

Dans les figures 28 à 35 qui suivent, est représentée une pièce intermédiaire transmettant le mouvement d'un actionneur à la paire d'organes d'entraînement pilotée par cet actionneur. La pièce intermédiaire représentée transmet le déplacement de l'actionneur à la paire d'organes d'entraînement, de manière à translater dans des sens opposés les deux organes d'entraînement de la paire d'organes d'entraînement, tout en maintenant sensiblement constant l'écartement entre les deux organes d'entraînement de la paire d'organes d'entraînement, de façon à faire tourner un organe médical souple allongé autour de lui-même lorsque celui-ci est disposé entre les deux organes d'entraînement de la paire d'organes d'entraînement. La pièce intermédiaire est une bascule transformant généralement une translation de l'actionneur selon une première direction en deux translations de sens opposés des deux organes respectifs d'entraînement selon une deuxième direction orthogonale à la première direction. Dans toutes ces figures, la double flèche H représente une translation horizontale dans les deux sens (aller et retour) tandis que la double flèche V représente une translation verticale dans les deux sens (aller et retour). Les sens horizontal et vertical sont par rapport à l'orientation des figures et ne préjugent pas du sens réel des actionneurs et de l'organe d'entraînement.

Dans la figure 28, la bascule comprend une platine 700 qui est reliée à l'actionneur et qui présente deux trous oblongs 701 et 702 inclinés d'inclinaison contraire dans lesquels coulissent au moins deux pions 703 et 704 respectivement reliés aux organes d'entraînement par l'intermédiaire respectivement des tiges 705 et 706, l'inclinaison des trous oblongs 701 et 702 étant plus proche de la direction horizontale que de la direction verticale. Par rapport à l'orientation de la figure, lorsque l'actionneur, situé sur la gauche, pousse la platine 700 vers la droite, la tige 705 monte tandis que la tige 706 descend, et lorsque l'actionneur pousse la platine 700 vers la gauche, la tige 706 monte tandis que la tige 705 descend.

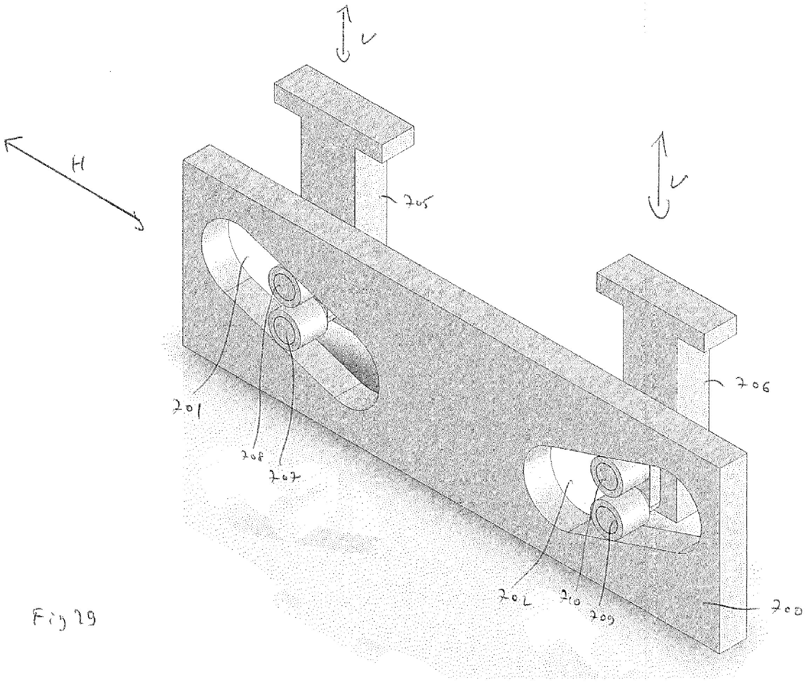

Dans la figure 29, la bascule la bascule comprend une platine 700 qui est reliée à l'actionneur et qui présente deux trous oblongs 701 et 702 inclinés d'inclinaison contraire dans lesquels coulissent au moins deux paires de galets, la première paire de galets 707 et 708 et la deuxième paire de galets 709 et 710, respectivement reliés aux organes d'entraînement par l'intermédiaire respectivement des tiges 705 et 706, l'inclinaison des trous oblongs 701 et 702 étant plus proche de la direction horizontale que de la direction verticale. Par rapport à l'orientation de la figure, lorsque l'actionneur, situé sur la gauche, pousse la platine 700 vers la droite, la tige 705 monte tandis que la tige 706 descend, et lorsque l'actionneur pousse la platine 700 vers la gauche, la tige 706 monte tandis que la tige 705 descend.

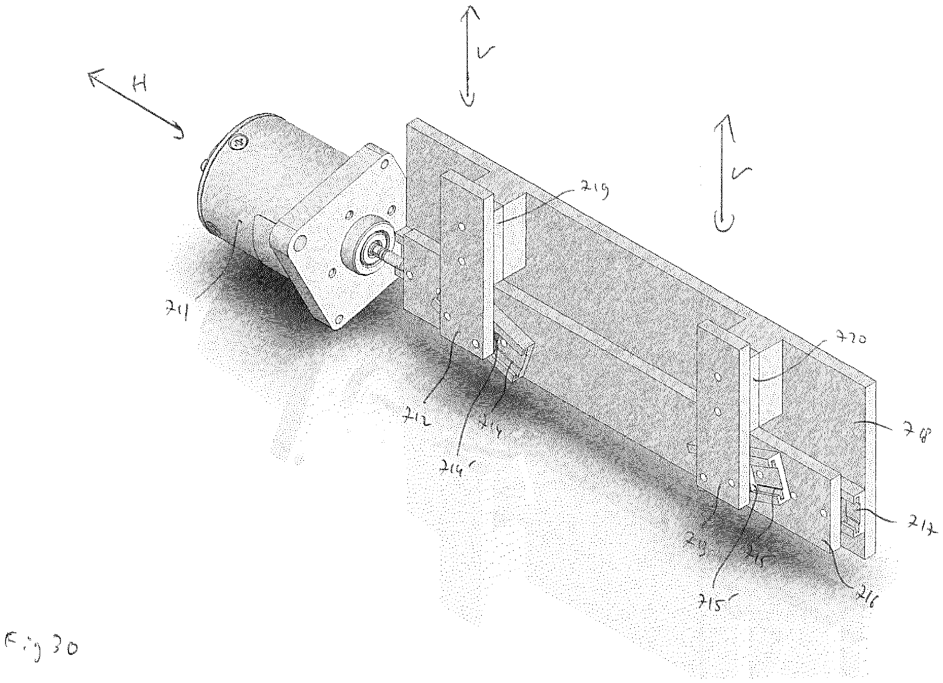

Dans la figure 30, la bascule comprend une platine 716 qui est reliée à l'actionneur 711 et qui présente deux rails obliques 714 et 715 inclinés d'inclinaison contraire dans lesquels coulissent au moins deux coulisseaux 714' et 715' respectivement reliés aux organes d'entraînement, l'inclinaison des rails 714 et 715 étant plus proche de la direction horizontale que de la direction verticale. Les deux rails 714 et 715 sont dans un même plan parallèle au plan formé par la direction horizontale et par la direction verticale. Par rapport à l'orientation de la figure, lorsque l'actionneur 711, situé sur la gauche, pousse la platine 716 vers la droite laquelle coulisse dans le rail 717 fixé sur le socle 718, la tige 712 monte tandis que la tige 713 descend, les tiges 712 et 713 étant verticalement guidés respectivement par les guides 719 et 720, et lorsque l'actionneur 711 pousse la platine 716 vers la gauche, la tige 713 monte tandis que la tige 712 descend.

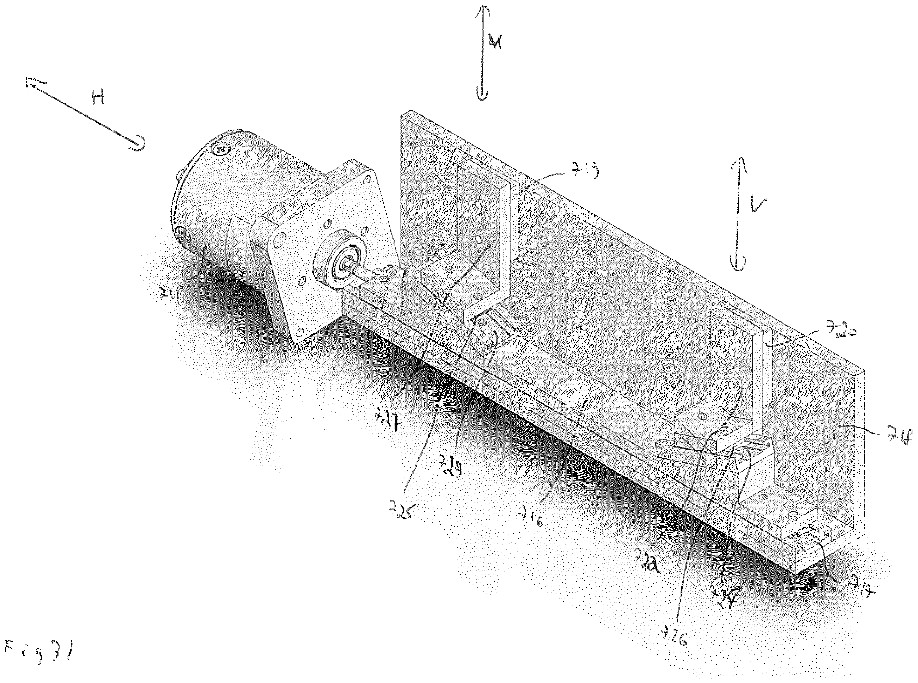

Dans la figure 31, la bascule comprend une platine 716 qui est reliée à l'actionneur 711 et qui présente deux rails obliques 723 et 724 inclinés d'inclinaison contraire dans lesquels coulissent au moins deux coulisseaux 725 et 726 respectivement reliés aux organes d'entraînement, l'inclinaison des rails 723 et 724 étant plus proche de la direction horizontale que de la direction verticale. Les deux rails 723 et 724 sont dans deux plans distincts perpendiculaires au plan formé par la direction horizontale et par la direction verticale. Par rapport à l'orientation de la figure, lorsque l'actionneur 711, situé sur la gauche, pousse la platine 716 vers la droite laquelle coulisse dans le rail 717 fixé sur le socle 718, la tige 721 monte tandis que la tige 722 descend, les tiges 721 et 722 étant verticalement guidés respectivement par les guides 719 et 720, et lorsque l'actionneur 711 pousse la platine 716 vers la gauche, la tige 722 monte tandis que la tige 721 descend. Les tiges 721 et 722 sont chacune essentiellement constituées de deux parties orthogonales entre elles.

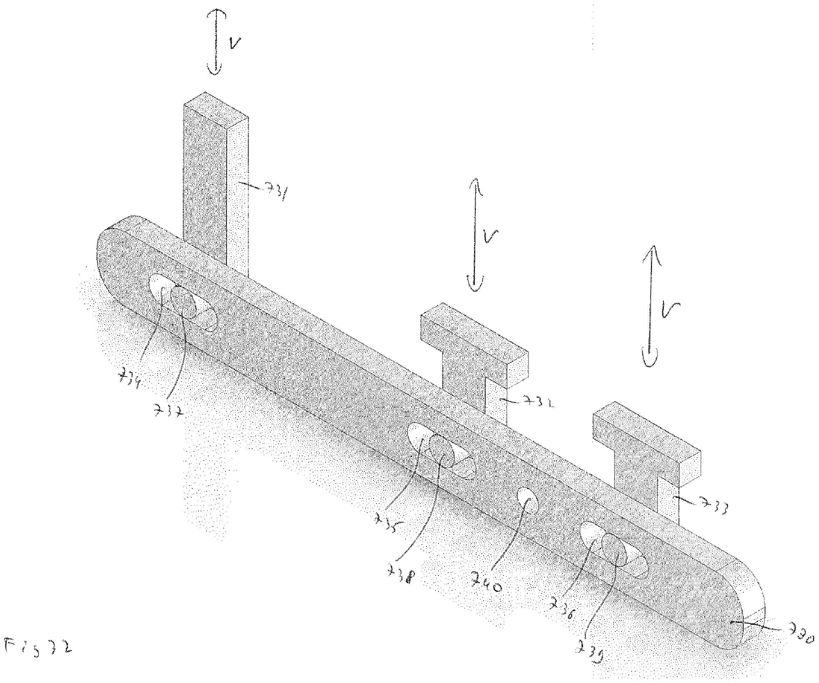

Dans la figure 32, la bascule est pivotante autour d'un axe 740 et comprend une platine 730 qui est reliée à l'actionneur et qui présente trois trous oblongs 734, 735 et 736, inclinés de même inclinaison dans lesquels coulissent au moins trois pions 737, 738 et 739, respectivement reliés à l'actionneur et aux organes d'entraînement, l'inclinaison des trous oblongs 734, 735 et 736, étant plus proche de la direction horizontale que de la direction verticale, deux des trous oblongs 735 et 736 étant disposés de manière symétrique par rapport à l'axe pivotant 740 et recevant les pions 738 et 739 reliés respectivement aux deux organes d'entraînement, le troisième trou oblong 734 étant disposé plus loin de l'axe pivotant 740 que les deux trous oblongs 738 et 739 reliés aux organes d'entraînement et recevant le pion 737 relié à l'actionneur. Lorsque la tige 731 de l'actionneur descend, la tige 732 du premier organe d'entraînement descend tandis que la tige 733 du deuxième organe d'entraînement monte. Lorsque la tige 731 de l'actionneur monte, la tige 732 du premier organe d'entraînement monte tandis que la tige 733 du deuxième organe d'entraînement descend.

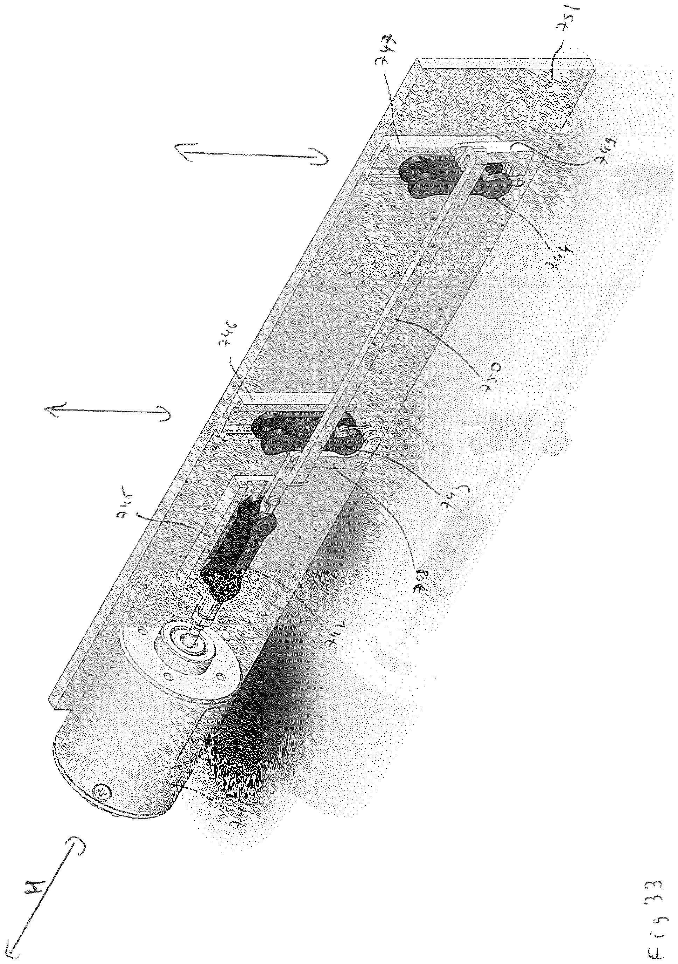

Dans la figure 33, la bascule comprend une platine 751 qui est reliée à l'actionneur 741 et qui présente deux systèmes à biellette 743 et 744 et manivelle 748 et 749 en forme de L, les deux manivelles 748 et 749 en forme de L étant orientées en sens contraire, la petite partie du L des manivelles 748 et 749 étant sensiblement selon la direction horizontale, la grande partie du L des manivelles 748 et 749 étant sensiblement selon la direction verticale. Les biellettes 743 et 744 sont respectivement reliées aux deux organes d'entraînement. Par rapport à l'orientation de la figure, lorsque l'actionneur 741, situé sur la gauche, pousse la biellette 742 actionnant elle-même la tringle 750 laquelle fait basculer les manivelles 748 et 749 en forme de L au niveau du coude du L, la biellette 743 monte dans le rail 746 fixé sur une platine 751 tandis que la biellette 744 descend dans le rail 747 fixé sur la platine 751. Inversement, lorsque l'actionneur 741, situé sur la gauche, tire la biellette 742 actionnant elle-même la tringle 750 laquelle fait basculer les manivelles 748 et 749 en forme de L au niveau du coude du L, la biellette 743 descend dans le rail 746 fixé sur une platine 751 tandis que la biellette 744 monte dans le rail 747 fixé sur la platine 751.

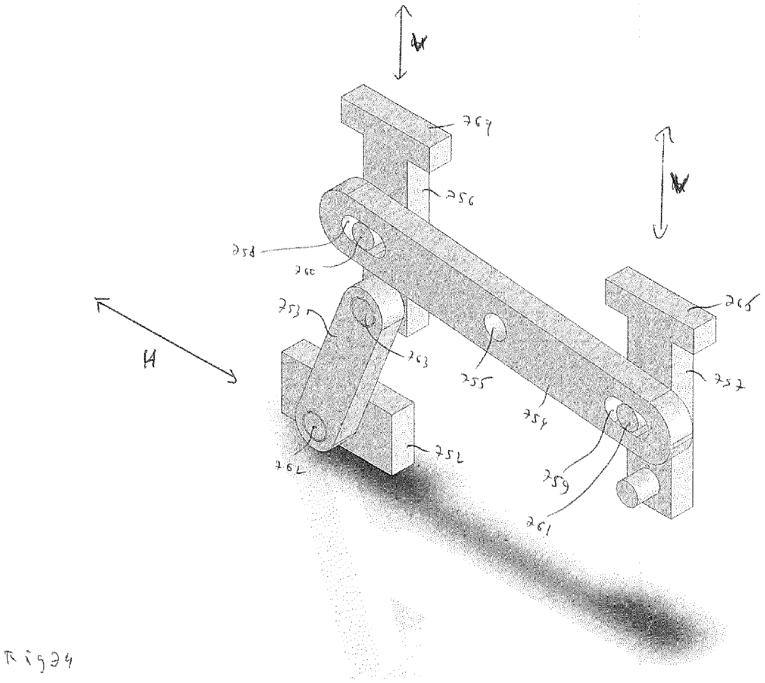

Dans la figure 34, la bascule comprend une platine 752 qui est reliée d'un côté à l'actionneur et qui est reliée de l'autre côté à une première extrémité 762 de bielle 753 dont la deuxième extrémité 763 est reliée à une première extrémité 763 d'une première tige 756 coulissant en son milieu 760 dans un premier trou oblong 758 situé à une première extrémité d'une barre 754 pivotant en son milieu 755 et dont la deuxième extrémité présente un deuxième trou oblong 759 dans lequel coulisse le milieu 761 d'une deuxième tige 757, les trous oblongs 758 et 759 étant parallèles à la barre 754, les deuxièmes extrémités 764 et 765 des deux tiges 756 et 757 étant respectivement reliées aux organes d'entraînement. Par rapport à l'orientation de la figure, lorsque l'actionneur, situé sur la gauche, pousse la platine 752, la bielle 753 fait monter la première tige 756, faisant ainsi basculer la barre 754 autour de son axe pivotant 755 dans le sens des aiguilles d'une montre, amenant alors la deuxième tige 757 à descendre. Inversement, lorsque l'actionneur, situé sur la gauche, tire la platine 752, la bielle 753 fait descendre la première tige 756, faisant ainsi basculer la barre 754 autour de son axe pivotant 755 dans le sens inverse des aiguilles d'une montre, amenant alors la deuxième tige 757 à monter.

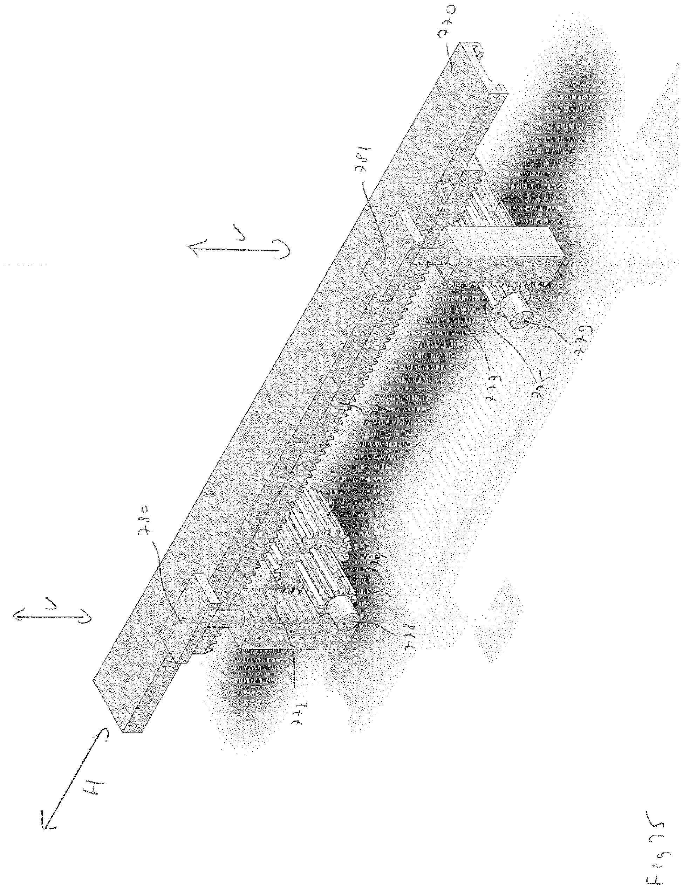

Dans la figure 35, la bascule comprend une platine 770 qui est reliée à l'actionneur et qui présente une première crémaillère 771 selon la direction horizontale, deux deuxièmes crémaillères 772 et 773 qui sont respectivement reliées aux organes d'entraînement et qui sont selon la direction verticale et dont les parties dentées se font face, deux systèmes d'engrenages situés entre la première crémaillère 771 et les deux deuxièmes crémaillères respectives 772 et 773, chacun des systèmes d'engrenage comprenant un grand engrenage 776 ou 777 engrenant avec la première crémaillère 771 et un petit engrenage 774 ou 775 engrenant avec une des deuxièmes crémaillères 772 ou 773. Le grand engrenage 776 et le petit engrenage 774 sont coaxiaux autour de l'axe 778. Le grand engrenage 777 et le petit engrenage 775 sont coaxiaux autour de l'axe 779. Par rapport à l'orientation de la figure, lorsque l'actionneur, situé sur la gauche, pousse la platine 770, poussant aussi vers la droite la première crémaillère 771, faisant tourner dans le sens des aiguilles d'une montre les grands engrenages 776 et 777, faisant également tourner dans le sens des aiguilles d'une montre les petits engrenages 774 et 775, faisant alors monter la deuxième crémaillère 772 et descendre la deuxième crémaillère 773, entraînant par conséquent la montée de la tige 780 et la descente de la tige 781, les deux tiges 780 et 781 étant respectivement reliées aux organes d'entraînement. Inversement, lorsque l'actionneur, situé sur la gauche, tire la platine 770, tirant aussi vers la gauche la première crémaillère 771, faisant tourner dans le sens inverse des aiguilles d'une montre les grands engrenages 776 et 777, faisant également tourner dans le sens inverse des aiguilles d'une montre les petits engrenages 774 et 775, faisant alors descendre la deuxième crémaillère 772 et monter la deuxième crémaillère 773, entraînant par conséquent la descente de la tige 780 et la montée de la tige 781.

## Revendications

1. Module robotisé d'entraînement d'organe médical souple allongé comprenant :
- une base (132),
- une paire (33) d'organes d'entraînement (24, 24') présentant chacun une surface d'entraînement (34, 34'), la paire (33) d'organes d'entraînement (24, 24') étant plaçable alternativement dans une configuration d'entraînement dans laquelle les surfaces d'entraînement (34, 34') des organes d'entraînement (24, 24') de la paire (33) d'organes d'entraînement (24, 24') sont en prise avec l'organe médical souple allongé à entraîner et disposés de part et d'autre de celui-ci, et dans une configuration libre dans laquelle la surface d'entraînement (34, 34') des organes d'entraînement (24, 24') de la paire (33) d'organes d'entraînement (24, 24') n'est pas en prise avec l'organe médical souple allongé,
la paire (33) d'organes d'entraînement (24, 24') étant montée mobile par rapport à la base (132) selon un degré de liberté entre une première et une deuxième positions,
- un organe de commande (18, 11) adapté pour commander de manière répétée cyclique un déplacement par rapport à la base (132) des organes d'entraînement (24, 24') de la paire (33) d'organes d'entraînement (24, 24') en configuration d'entraînement de la première à la deuxième position, entraînant ainsi l'organe médical souple allongé par rapport à la base (132), et un déplacement par rapport à la base (132) des organes d'entraînement (24, 24') de la paire (33) d'organes d'entraînement (24, 24') en configuration libre de la deuxième à la première position sans entraîner l'organe médical souple allongé par rapport à la base,
dans lequel le degré de liberté entre les première et deuxième positions est un premier degré de liberté, dans lequel la paire (33) d'organes d'entraînement (24, 24') est également montée mobile par rapport à la base (132) selon un deuxième degré de liberté différant du premier degré de liberté entre la première et une troisième positions,
l'organe de commande (18, 11) étant adapté pour commander de manière répétée cyclique un déplacement par rapport à la base (132) des organes d'entraînement (24, 24') en configuration d'entraînement de la première à la troisième position, entraînant ainsi l'organe médical souple allongé par rapport à la base (132), et un déplacement par rapport à la base (132) des organes d'entraînement (24, 24') en configuration libre de la troisième à la première position sans entraîner l'organe médical souple par rapport à la base (132),
et **caractérisé en ce que** la translation des organes d'entraînement (24, 24') par rapport à la base (132) selon une direction (Z) transverse à la direction longitudinale (X) locale de l'organe médical souple allongé et en des sens opposés est adaptée pour permettre un roulement de l'organe médical souple allongé sur les surfaces d'entraînement (34, 34') autour de la direction longitudinale (X) locale de l'organe médical souple allongé.

2. Module robotisé selon la revendication 1, dans lequel le déplacement par rapport à la base (132) des organes d'entraînement (24, 24') de la première position à la deuxième position et le déplacement par rapport à la base (132) des organes d'entraînement (24, 24') de la première à la troisième position comprennent deux combinaisons distinctes parmi :
- une translation des organes d'entraînement (24, 24') par rapport à la base (132) selon une direction parallèle à la direction longitudinale (X) locale de l'organe médical souple allongé,
- une translation des organes d'entraînement (24, 24') par rapport à la base (132) selon une direction transversale (Z) à la direction longitudinale (X) locale de l'organe médical souple allongé et en des sens opposés,
- une translation des organes d'entraînement (24, 24') par rapport à la base (132) selon une direction (Z) transverse à la direction longitudinale (X) locale de l'organe médical souple allongé et en un même sens,
- une translation des organes d'entraînement (24, 24') par rapport à la base (132) selon une direction transverse (Y) à la direction longitudinale (X) locale de l'organe médical souple allongé et en un même sens.

3. Module robotisé d'entraînement selon l'une des revendications 1 à 2, dans lequel la paire (33) d'organes d'entraînement (24, 24') est plaçable de sa configuration libre à sa configuration d'entraînement par un déplacement relatif des organes d'entraînement (24, 24') par rapport à la base (132), et de préférence dans lequel les première et deuxième positions définissent un premier degré de liberté, dans lequel la paire d'organes d'entraînement est également montée mobile par rapport à la base selon un troisième degré de liberté de sa configuration libre à sa configuration d'entraînement.

4. Module robotisé d'entraînement selon l'une quelconque des revendications 1 à 3, dans lequel la base (132) est une première base, la paire (33) d'organes d'entraînement (24, 24') est une première paire d'organes d'entraînement, le module robotisé comprenant en outre :
- une deuxième base (132'),
- une deuxième paire (33') d'organes d'entraînement (24", 24‴) présentant chacun une surface d'entraînement (34", 34‴), la deuxième paire (33') d'organes d'entraînement (24", 24‴) étant plaçable alternativement dans une configuration d'entraînement dans laquelle les surfaces d'entraînement (34", 34‴) des organes d'entraînement (24", 24‴) de la deuxième paire (33') d'organes d'entraînement (24", 24‴) sont en prise avec l'organe médical souple allongé à entraîner et disposés de part et d'autre de celui-ci, et dans une configuration libre dans laquelle la surface d'entraînement (34'', 34''') des organes d'entraînement (24", 24‴) de la deuxième paire (33') d'organes d'entraînement (24", 24‴) n'est pas en prise avec l'organe médical souple allongé,
la deuxième paire (33') d'organes d'entraînement (24", 24‴) étant montée mobile par rapport à la deuxième base (132') selon un degré de liberté entre une première et une deuxième positions,
- l'organe de commande (18, 11) étant adapté en outre pour commander de manière répétée cyclique un déplacement par rapport à la base (132') des organes d'entraînement (24", 24") de la deuxième paire (33') d'organes d'entraînement (24", 24‴) en configuration d'entraînement de la première à la deuxième position, entraînant ainsi l'organe médical souple allongé par rapport à la deuxième base (132'), et un déplacement par rapport à la deuxième base (132') des organes d'entraînement (24", 24") de la deuxième paire (33') d'organes d'entraînement (24", 24‴) en configuration libre de la deuxième à la première position sans entraîner l'organe médical souple par rapport à la deuxième base (32').

5. Module robotisé d'entraînement selon la revendication 4, dans lequel la première base (132) et la deuxième base (132') sont solidaires ou communes.

6. Module robotisé d'entraînement selon la revendication 4 ou 5, dans lequel l'organe de commande (18, 11) est adapté pour commander les déplacements des organes d'entraînement de la première paire et de la deuxième paire de manière synchronisée.

7. Module robotisé d'entraînement selon la revendication 6, dans lequel l'organe de commande (18, 11) est adapté pour placer les organes d'entraînement de la première paire et de la deuxième paire simultanément en configuration d'entraînement, et/ou dans lequel l'organe de commande (18, 11) est adapté pour placer les organes d'entraînement de la première paire et de la deuxième paire simultanément en configuration libre, et/ou dans lequel l'organe de commande (18, 11) est adapté pour placer simultanément les organes d'entraînement de la première paire et de la deuxième paire pour les uns en configuration d'entraînement et pour les autres en configuration libre.

8. Module robotisé d'entraînement d'un élément mobile sous forme d'organe médical souple allongé selon l'une quelconque des revendications 1 à 7, **caractérisé en ce qu'**il comprend une chaîne de transmission de mouvement comprenant :
- un socle (640) d'organe d'entraînement (24, 24') d'un élément mobile (15', 15"),
- trois actionneurs (610, 620, 630) pilotant le socle (640) de l'organe d'entraînement (24, 24') respectivement selon trois directions (Y, X, Z) de translation distinctes entre elles, par l'intermédiaire de trois interfaces (612, 622, 632) respectives avec le socle (640) de l'organe d'entraînement (24, 24'),
et **en ce que** l'intersection des surfaces moyennes des trois interfaces (612, 622, 632) est localisée en région centrale du socle (640) de l'organe d'entraînement (24, 24').

9. Module robotisé d'entraînement d'un élément mobile sous forme d'organe médical souple allongé selon la revendication 8, caractérisé en en ce que :
- les trois interfaces (612, 622, 632) sont sensiblement planes,
- ces trois interfaces (612, 622, 632) sont orthogonales les unes aux autres,
- et ces trois interfaces (612, 622, 632) sont imbriquées les unes dans les autres.

10. Module robotisé d'entraînement selon l'une quelconque des revendications précédentes, **caractérisé en ce qu'**il comprend aussi au moins :
- un actionneur (711, 741) pilotant une paire d'organes d'entraînement,
- une pièce intermédiaire transmettant le déplacement de l'actionneur (711, 741) à la paire d'organes d'entraînement, de manière à translater dans des sens opposés les deux organes d'entraînement de la paire d'organes d'entraînement, tout en maintenant sensiblement constant l'écartement entre les deux organes d'entraînement de la paire d'organes d'entraînement, de façon à faire tourner un organe médical souple allongé autour de lui-même lorsque celui-ci est disposé entre les deux organes d'entraînement de la paire d'organes d'entraînement.

11. Module robotisé d'entraînement selon la revendication 10, **caractérisé en ce que** :
- la pièce intermédiaire est une bascule transformant une translation de l'actionneur (711, 741) selon une première direction (H) en deux translations de sens opposés des deux organes respectifs d'entraînement selon une deuxième direction orthogonale (V) à la première direction (H).

12. Module robotisé d'entraînement selon la revendication 11, **caractérisé en ce que** :
- la bascule comprend une platine (700) qui est reliée à l'actionneur et qui présente deux trous oblongs (701, 702) inclinés d'inclinaison contraire dans lesquels coulissent au moins deux pions (703, 704) respectivement reliés aux organes d'entraînement, l'inclinaison des trous oblongs (701, 702) étant plus proche de la première direction (H) que de la deuxième direction (V).

13. Module robotisé d'entraînement selon la revendication 11, **caractérisé en ce que** :
- la bascule comprend une platine (700) qui est reliée à l'actionneur et qui présente deux trous oblongs (701, 702) inclinés d'inclinaison contraire dans lesquels coulissent au moins deux galets (707, 708, 709, 710) respectivement reliés aux organes d'entraînement, l'inclinaison des trous oblongs (701, 702) étant plus proche de la première direction (H) que de la deuxième direction (V).

14. Module robotisé d'entraînement selon la revendication 11, **caractérisé en ce que** :
- la bascule comprend une platine (716) qui est reliée à l'actionneur (711) et qui présente deux rails obliques (714, 715) inclinés d'inclinaison contraire dans lesquels coulissent au moins deux coulisseaux (714', 715') respectivement reliés aux organes d'entraînement, l'inclinaison des rails (714, 715) étant plus proche de la première direction (H) que de la deuxième direction (V).

15. Module robotisé d'entraînement selon la revendication 14, **caractérisé en ce que** :
- les deux rails (714, 715) sont dans un même plan parallèle au plan formé par la première direction (H) et par la deuxième direction (V).

16. Module robotisé d'entraînement selon la revendication 14, **caractérisé en ce que** :
- les deux rails (723, 724) sont dans deux plans distincts perpendiculaires au plan formé par la première direction (H) et par la deuxième direction (V).

17. Module robotisé d'entraînement selon la revendication 10, **caractérisé en ce que** :
- la bascule est pivotante autour d'un axe (740) et comprend une platine (730) qui est reliée à l'actionneur et qui présente trois trous oblongs (734, 735, 736) inclinés de même inclinaison dans lesquels coulissent au moins trois pions (737, 738, 739) ou trois galets respectivement reliés à l'actionneur et aux organes d'entraînement, l'inclinaison des trous oblongs (734, 735, 736) étant plus proche de la première direction (H) que de la deuxième direction (V), deux des trous oblongs (735, 736) étant disposés de manière symétrique par rapport à l'axe pivotant (740) et recevant les pions (738, 739) ou les galets reliés respectivement aux deux organes d'entraînement, le troisième trou oblong (734) étant disposé plus loin de l'axe pivotant (740) que les deux trous oblongs (735, 736)reliés aux organes d'entraînement et recevant le pion (737) ou le galet relié à l'actionneur.

18. Module robotisé d'entraînement selon la revendication 11, **caractérisé en ce que** :
- la bascule comprend une platine (751) qui est reliée à l'actionneur (741) et qui présente deux systèmes à biellette (743, 744) et manivelle (748, 749) en forme de L, les deux manivelles (748, 749) en forme de L étant orientées en sens contraire, la petite partie du L des manivelles (748, 749) étant sensiblement selon la première direction (H), la grande partie du L des manivelles (748, 749) étant sensiblement selon la deuxième direction (V).

19. Module robotisé d'entraînement selon la revendication 11, **caractérisé en ce que** :
- la bascule comprend une platine (752) qui est reliée d'un côté à l'actionneur et qui est reliée de l'autre côté à une première extrémité (762) de bielle (753) dont la deuxième extrémité (763) est reliée à une première extrémité (763) d'une première tige (756) coulissant en son milieu (760) dans un premier trou oblong (758) situé à une première extrémité d'une barre (754) pivotant en son milieu (755) et dont la deuxième extrémité présente un deuxième trou oblong (759) dans lequel coulisse le milieu (761) d'une deuxième tige (757), les trous oblongs (758, 759) étant parallèles à la barre (754), les deuxièmes extrémités (764, 765) des deux tiges (756, 757) étant respectivement reliées aux organes d'entraînement.

20. Module robotisé d'entraînement selon la revendication 11, **caractérisé en ce que** :
- la bascule comprend une platine (770) qui est reliée à l'actionneur et qui présente une première crémaillère (771) selon la première direction (H), deux deuxièmes crémaillères (772, 773) qui sont respectivement reliées aux organes d'entraînement et qui sont selon la deuxième direction (V) et dont les parties dentées se font face, deux systèmes d'engrenages situés entre la première crémaillère (771) et les deux deuxièmes crémaillères (772, 773) respectives, chacun des systèmes d'engrenage comprenant un grand engrenage (776, 777) engrenant avec la première crémaillère (771) et un petit engrenage (774, 775) engrenant avec une des deuxièmes crémaillères (772, 773).

## Patentansprüche

1. Robotisiertes Antriebsmodul eines länglichen flexiblen medizinischen Elements, umfassend:
- eine Basis (132),
- ein Paar (33) von Antriebselementen (24, 24'), welche jeweils eine Antriebsfläche (34, 34') aufweisen, wobei das Paar (33) von Antriebselementen (24, 24') alternativ in einer Antriebskonfiguration, in welcher die Antriebsflächen (34, 34') der Antriebselemente (24, 24') des Paars (33) von Antriebselementen (24, 24') mit dem anzutreibenden länglichen flexiblen medizinischen Element eingreifen und beiderseits davon angeordnet sind, und in einer freien Konfiguration platzierbar sind, in welcher die Antriebsfläche (34, 34') der Antriebselemente (24, 24') des Paars (33) von Antriebselementen (24, 24') nicht mit dem länglichen flexiblen medizinischen Element eingreifen,
wobei das Paar (33) von Antriebselementen (24, 24') bezüglich der Basis (132) gemäß einem Freiheitsgrad zwischen einer ersten und einer zweiten Position bewegbar montiert ist,
- ein Steuerelement (18, 11), welches dazu eingerichtet ist, in zyklisch wiederholter Weise eine Verlagerung bezüglich der Basis (132) der Antriebselemente (24, 24') des Paars (33) von Antriebselementen (24, 24') in Antriebskonfiguration von der ersten zu der zweiten Position zu steuern, wodurch das längliche flexible medizinische Element bezüglich der Basis (132) angetrieben wird, sowie eine Verlagerung bezüglich der Basis (132) der Antriebselemente (24, 24') des Paars (33) von Antriebselementen (24, 24') in freier Konfiguration von der zweiten zu der ersten Position, ohne das längliche flexible medizinische Element bezüglich der Basis anzutreiben,
wobei der Freiheitsgrad zwischen den ersten und zweiten Positionen ein erster Freiheitsgrad ist, wobei das Paar (33) von Antriebselementen (24, 24') ferner bezüglich der Basis (132) gemäß einem zweiten Freiheitsgrad, welcher verschieden von dem ersten Freiheitsgrad ist, zwischen der ersten und einer dritten Position bewegbar montiert ist,
wobei das Steuerelement (18, 11) dazu eingerichtet ist, in zyklisch wiederholter Weise eine Verlagerung bezüglich der Basis (132) der Antriebselemente (24, 24') in Antriebskonfiguration von der ersten zu der dritten Position zu steuern, wodurch das längliche flexible medizinische Element bezüglich der Basis (132) angetrieben wird, sowie eine Verlagerung bezüglich der Basis (132) der Antriebselemente (24, 24') in freier Konfiguration von der dritten zu der ersten Position, ohne das flexible medizinische Element bezüglich der Basis (132) anzutreiben,
**dadurch gekennzeichnet, dass** die Verlagerung der Antriebselemente (24, 24') bezüglich der Basis (132) gemäß einer Richtung (Z) transversal zu der lokalen longitudinalen Richtung (X) des länglichen flexiblen medizinischen Elements und in umgekehrtem Sinn dazu eingerichtet ist, ein Rollen des länglichen flexiblen medizinischen Elements auf den Antriebsflächen (34, 34') um die lokale longitudinale Richtung (X) des länglichen flexiblen medizinischen Elements zu erlauben.

2. Robotisiertes Modul nach Anspruch 1, wobei die Verlagerung bezüglich der Basis (132) der Antriebselemente (24, 24') von der ersten Position zu der zweiten Position und die Verlagerung bezüglich der Basis (132) der Antriebselemente (24, 24') von der ersten zu der dritten Position zwei unterschiedliche Kombinationen umfasst, aus:
- einer Verlagerung der Antriebselemente (24, 24') bezüglich der Basis (132) gemäß einer Richtung parallel zu der lokalen longitudinalen Richtung (X) des länglichen flexiblen medizinischen Elements,
- einer Verlagerung der Antriebselemente (24, 24') bezüglich der Basis (132) gemäß einer Richtung (Z) transversal zu der lokalen longitudinalen Richtung (X) des länglichen flexiblen medizinischen Elements und in entgegengesetzten Sinnen,
- eine Verlagerung der Antriebselemente (24, 24') bezüglich der Basis (132) gemäß einer Richtung (Z) transversal zu der lokalen longitudinalen Richtung (X) des länglichen flexiblen medizinischen Elementes und in einem selben Sinn,
- eine Verlagerung der Antriebselemente (24, 24') bezüglich der Basis (132) gemäß einer Richtung (Y) transversal zu der lokalen longitudinalen Richtung (X) des länglichen flexiblen medizinischen Elements und in einem selben Sinn.

3. Robotisiertes Antriebsmodul nach einem der Ansprüche 1 bis 2, wobei das Paar (33) von Antriebselementen (24, 24') von seiner freien Konfiguration zu seiner Antriebskonfiguration durch ein relatives Verlagern der Antriebselemente (24, 24') bezüglich der Basis (132) platzierbar ist, und wobei vorzugsweise die ersten und zweiten Positionen einen ersten Freiheitsgrad definieren, wobei das Paar von Antriebselementen ferner bewegbar bezüglich der Basis gemäß einem dritten Freiheitsgrad von seiner freien Konfiguration zu seiner Antriebskonfiguration montiert ist.

4. Robotisiertes Antriebsmodul nach einem der Ansprüche 1 bis 3, wobei die Basis (132) eine erste Basis ist, das Paar (33) von Antriebselementen (24, 24') ein erstes Paar von Antriebselementen ist, wobei das robotisierte Modul ferner umfasst:
- eine zweite Basis (132'),
- ein zweites Paar (33') von Antriebselementen (24", 24‴), welche jeweils eine Antriebsfläche (34", 34‴) aufweisen, wobei das zweite Paar (33') von Antriebselementen (24", 24‴) alternativ in einer Antriebskonfiguration, in welcher die Antriebsflächen (34", 34‴) der Antriebselemente (24", 24‴) des zweiten Paars (33') von Antriebselementen (24", 24‴) mit dem anzutreibenden länglichen flexiblen medizinischen Element eingreifen und beiderseits davon angeordnet sind, und in einer freien Konfiguration platzierbar sind, in welcher die Antriebsfläche (34", 34‴) der Antriebselemente (24", 24‴) des zweiten Paars (33') von Antriebselementen (24", 24‴) nicht mit dem länglichen flexiblen medizinischen Element eingreift,
wobei das zweite Paar (33') von Antriebselementen (24", 24‴) bezüglich der zweiten Basis (132') gemäß einem Freiheitsgrad zwischen einer ersten und einer zweiten Position bewegbar montiert ist,
- wobei das Steuerelement (18,11) ferner dazu eingerichtet ist, in zyklisch wiederholter Weise eine Verlagerung bezüglich der Basis (132') der Antriebselemente (24", 24‴) des zweiten Paars (33') von Antriebselementen (24", 24‴) in Antriebskonfiguration von der ersten zu der zweiten Position zu steuern, wodurch das längliche flexible medizinische Element bezüglich der zweiten Basis (132') angetrieben wird, sowie eine Verlagerung bezüglich der zweiten Basis (132') der Antriebselemente (24", 24‴) des zweiten Paars (33') von Antriebselementen (24", 24‴) in freier Konfiguration von der zweiten zu der ersten Position, ohne das flexible medizinische Element bezüglich der zweiten Basis (32') anzutreiben.

5. Robotisiertes Antriebsmodul nach Anspruch 4, wobei die erste Basis (132) und die zweite Basis (132') verbunden oder gemeinsam sind.

6. Robotisiertes Antriebsmodul nach Anspruch 4 oder 5, wobei das Steuerelement (18, 11) dazu eingerichtet ist, die Verlagerungen der Antriebselemente des ersten Paars und des zweiten Paars in synchronisierter Weise zu steuern.

7. Robotisiertes Antriebsmodul nach Anspruch 6, wobei das Steuerelement (18, 11) dazu eingerichtet ist, die Antriebselemente des ersten Paars und des zweiten Paars gleichzeitig in Antriebskonfiguration zu platzieren und/oder wobei das Steuerelement (18,11) dazu eingerichtet ist, die Antriebseinheiten des ersten Paars und des zweiten Paars gleichzeitig in freie Konfiguration zu platzieren, und/oder wobei das Antriebselement (18, 11) dazu eingerichtet ist, die Antriebselemente des ersten Paars und des zweiten Paares gleichzeitig einerseits in Antriebskonfiguration und andererseits in freie Konfiguration zu platzieren.

8. Robotisiertes Antriebsmodul eines bewegbaren Elements in Form eines länglichen flexiblen medizinischen Elements nach einem der Ansprüche 1 bis 7, **dadurch gekennzeichnet, dass** es eine Bewegung-Übertragungskette umfasst, umfassend:
- einen Sockel (640) eines Antriebselements (24, 24') eines bewegbaren Elements (15', 15"),
- drei Aktuatoren (610, 620, 630), welche den Sockel (640) des Antriebselements (24, 24') jeweils bezüglich dreier untereinander verschiedener Verlagerungsrichtungen (Y, X, Z) mittels dreier jeweiliger Schnittstellen (612, 622, 632) mit dem Sockel (640) des Antriebselements (24, 24') führen,
und dadurch, dass der Schnittpunkt der Mittelflächen der drei Schnittstellen (612, 622, 632) in einem zentralen Bereich des Sockels (640) des Antriebselements (24, 24') angeordnet ist.

9. Robotisiertes Antriebsmodul eines bewegbaren Elements in Form eines länglichen flexiblen medizinischen Elements nach Anspruch 8, **dadurch gekennzeichnet, dass**:
- die drei Schnittstellen (612, 622, 632) im Wesentlichen eben sind,
- die drei Schnittstellen (612, 622, 632) untereinander orthogonal sind,
- und die drei Schnittstellen (612, 622, 632) miteinander verkeilt sind.

10. Robotisiertes Antriebsmodul nach einem der vorhergehenden Ansprüche, **dadurch gekennzeichnet, dass** es ebenfalls wenigstens umfasst:
- einen Aktuator (711, 741), welcher ein Paar von Antriebselementen führt,
- ein Zwischenstück, welches die Verlagerung des Aktuators (711, 741) an das Paar von Antriebselementen derart überträgt, dass die beiden Antriebselemente des Paars von Antriebselementen in entgegengesetzte Sinne verlagert werden, während der Abstand zwischen den beiden Antriebselementen des Paars von Antriebselementen im Wesentlichen konstant gehalten wird, in der Weise, dass ein längliches flexibles medizinisches Element dazu gebracht wird, sich um sich selbst zu drehen, wenn es zwischen den beiden Antriebselementen des Paars von Antriebselementen angeordnet ist.

11. Robotisiertes Antriebsmodul nach Anspruch 10, **dadurch gekennzeichnet, dass**:
- das Zwischenstück eine Wippe ist, welche eine Verlagerung des Aktuators (711, 741) gemäß einer ersten Richtung (H) in zwei Verlagerungen in entgegengesetzte Sinne der beiden entsprechenden Antriebselemente gemäß einer zweiten Richtung (V) orthogonal zu der ersten Richtung (H) umwandelt.

12. Robotisiertes Antriebsmodul nach Anspruch 11, **dadurch gekennzeichnet, dass**:
- die Wippe eine Platte (700) umfasst, welche mit dem Aktuator verbunden ist und welche zwei Langlöcher (701, 702) aufweist, welche mit entgegengesetzter Neigung geneigt sind, in welchen wenigstens zwei Stifte (703, 704) laufen, welche jeweils mit den Antriebselementen verbunden sind, wobei die Neigung der Langlöcher (701, 702) näher zu der ersten Richtung (H) als zu der zweiten Richtung (V) ist.

13. Robotisiertes Antriebsmodul nach Anspruch 11, **dadurch gekennzeichnet, dass**:
- die Wippe eine Platte (700) umfasst, welche mit dem Aktuator verbunden ist, und welche zwei Langlöcher (701, 702) aufweist, welche mit entgegengesetzter Neigung geneigt sind, in welchen wenigstens zwei Rollen (707, 708, 709, 710) laufen, welche jeweils mit den Antriebselementen verbunden sind, wobei die Neigung der Langlöcher (701, 702) näher zu der ersten Richtung (H) als zu der zweiten Richtung (V) ist.

14. Robotisiertes Antriebsmodul nach Anspruch 11, **dadurch gekennzeichnet, dass**:
- die Wippe eine Platte (716) umfasst, welche mit dem Aktuator (711) verbunden ist und welche zwei schräge Schienen (714, 715) aufweist, welche mit entgegengesetzter Neigung geneigt sind, in welchen wenigstens zwei Läufer (714', 715') laufen, welche jeweils mit den Antriebselementen verbunden sind, wobei die Neigung der Schienen (714, 715) näher zu der ersten Richtung (H) als zu der zweiten Richtung (V) ist.

15. Robotisiertes Antriebsmodul nach Anspruch 14, **dadurch gekennzeichnet, dass**:
- die beiden Schienen (714, 715) in einer selben Ebene parallel zu der Ebene liegen, welche durch die erste Richtung (H) und durch die zweite Richtung (V) gebildet ist.

16. Robotisiertes Antriebsmodul nach Anspruch 14, **dadurch gekennzeichnet, dass**:
- die beiden Schienen (723, 724) in zwei verschiedenen Ebenen liegen, welche senkrecht zu der Ebene sind, welche durch die erste Richtung (H) und durch die zweiten Richtung (V) gebildet ist.

17. Robotisiertes Antriebsmodul nach Anspruch 10, **dadurch gekennzeichnet, dass**:
- die Wippe um eine Achse (740) schwenkbar ist und eine Platte (730) umfasst, welche mit dem Aktuator verbunden ist und welche drei Langlöcher (734, 735, 736) aufweist, welche mit gleicher Neigung geneigt sind, in welchen wenigstens drei Stifte (737, 738, 739) oder drei Rollen laufen, welche jeweils mit dem Aktuator und den Antriebselementen verbunden sind, wobei die Neigung der drei Langlöcher (734, 735, 736) näher zu der ersten Richtung (H) als zu der zweiten Richtung (V) ist, wobei zwei der Langlöcher (735, 736) in symmetrischer Weise bezüglich der Schwenkachse (740) angeordnet sind und die Stifte (738, 739) oder die Rollen aufnehmen, welche jeweils mit den beiden Antriebselementen verbunden sind, wobei das dritte Langloch (734) näher zu der Schwenkachse (740) angeordnet ist als die beiden Langlöcher (735, 736), welche mit den Antriebselementen verbunden sind und den Stift (737) oder die Rolle aufnehmen, welche/r mit dem Aktuator verbunden ist.

18. Robotisiertes Antriebsmodul nach Anspruch 11, **dadurch gekennzeichnet, dass**:
- die Wippe eine Platte (751) umfasst, welche mit dem Aktuator (741) verbunden ist und welche zwei Systeme mit Gestänge (743, 744) und Kurbel (748, 749) in L-Form aufweist, wobei die beiden Kurbeln (748, 749) in L-Form in entgegengesetztem Sinn orientiert sind, wobei der kleine Teil des L der Kurbeln (748, 749) im Wesentlichen gemäß der ersten Richtung (H) liegt, wobei der große Teil des L der Kurbeln (748, 749) im Wesentlichen entlang der zweiten Richtung (V) liegt.

19. Robotisiertes Antriebsmodul nach Anspruch 11, **dadurch gekennzeichnet, dass**:
- die Wippe eine Platte (752) umfasst, welche an einer Seite mit dem Aktuator verbunden ist und welche an der anderen Seite mit einem ersten Ende (762) einer Stange (753) verbunden ist, deren zweites Ende (763) mit einem ersten Ende (763) eines ersten Stabs (756) verbunden ist, welcher an seiner Mitte (760) in einem ersten Langloch (758) läuft, welches an einem ersten Ende eines Balkens (754) angeordnet ist, welcher an seiner Mitte (755) schwenkbar ist, und dessen zweites Ende ein zweites Langloch (759) aufweist, in welchem die Mitte (761) eines zweiten Stabs (757) läuft, wobei die Langlöcher (758, 759) parallel zu dem Balken (754) sind, wobei die zweite Enden (764, 765) der beiden Stäbe (756, 757) jeweils mit den Antriebselementen verbunden sind.

20. Robotisiertes Antriebsmodul nach Anspruch 11, **dadurch gekennzeichnet, dass**:
- die Wippe eine Platine (770) umfasst, welche mit dem Aktuator verbunden ist und welche aufweist: ein erstes Zahngestänge (771) gemäß der ersten Richtung (H), zwei zweite Zahngestänge (772, 773), welche jeweils mit den Antriebselementen verbunden sind und welche gemäß der zweiten Richtung (V) liegen und deren Zahnabschnitte einander gegenüberstehen, zwei Räderwerksysteme, welche jeweils zwischen dem ersten Zahngestänge (771) und den beiden zweiten Zahngestängen (772, 773) angeordnet sind, wobei jedes der Räderwerksysteme ein großes Räderwerk (776, 777), welches mit dem ersten Zahngestänge (771) eingreift, und ein kleines Räderwerk (774, 775) umfasst, welches mit einem der zweiten Zahngestänge (772, 773) eingreift.

## Claims

1. A robotised module for actuating an elongated flexible medical device comprising:
- a base (132),
- a pair (33) of actuation members (24, 24') each having an actuation surface (34, 34'), the pair (33) of actuation members (24, 24') being suitable for being placed alternately in an actuation configuration wherein the actuation surfaces (34, 34') of the actuation members (24, 24') of the pair (33) of actuation members (24, 24') are engaged with the elongated flexible medical device to be actuated and arranged on either side thereof, and in a free configuration wherein the actuation surface (34, 34') of the actuation members (24, 24') of the pair (33) of actuation members (24, 24') is not engaged with the elongated flexible medical device,
the pair (33) of actuation devices (24, 24') being movably mounted with respect to the base (132) according to a degree of freedom between a first and a second positions,
- a control member (18, 11) suitable for controlling in a cyclically repeated manner a movement with respect to the base (132) of the actuation members (24, 24') of the pair (33) of actuation members (24, 24') in the actuation configuration from the first to the second position, thus actuating the elongated flexible medical device with respect to the base (132), and a movement with respect to the base (132) of the actuation members (24, 24') of the pair (33) of actuation members (24, 24') in the free configuration from the second to the first position without actuating the elongated flexible medical device with respect to the base,
wherein the degree of freedom between the first and second positions is a first degree of freedom, wherein the pair (33) of actuation members (24, 24') is also movably mounted with respect to the base (132) according to a second degree of freedom differing from the first degree of freedom between the first and a third positions,
the control member (18, 11) being suitable for controlling in a cyclically repeated manner a movement with respect to the base (132) of the actuation members (24, 24') in the actuation configuration from the first to the third position, thus actuating the elongated flexible medical device with respect to the base (132), and a movement with respect to the base (132) of the actuation members (24, 24') in the free configuration from the third to the first position without actuating the flexible medical device with respect to the base (132),
**characterised in that** the translation of the actuation members (24, 24') with respect to the base (132) along a direction (Z) transverse to the local longitudinal direction (X) of the elongated flexible medical device and in opposite directions is suitable for enabling rolling of the elongated flexible medical device on the actuation surfaces (34, 34') about the local longitudinal direction (X) of the elongated flexible medical device.

2. The robotised module according to claim 1, wherein the movement with respect to the base (132) of the actuation members (24, 24') from the first position to the second position and the movement with respect to the base (132) of the actuation members (24, 24') from the first to the third position comprise two distinct combinations of:
- a translation of the actuation members (24, 24') with respect to the base (132) along a direction parallel to the local longitudinal direction (X) of the elongated flexible medical device,
- a translation of the actuation members (24, 24') with respect to the base (132) along a direction (Z) transverse to the local longitudinal direction (X) of the elongated flexible medical device and in opposite directions,
- a translation of the actuation members (24, 24') with respect to the base (132) along a direction (Z) transverse to the local longitudinal direction (X) of the elongated flexible medical device and in the same direction,
- a translation of the actuation members (24, 24') with respect to the base (132) along a direction (Y) transverse to the local longitudinal direction (X) of the elongated flexible medical device and in the same direction.

3. The robotised actuation module according to one of claims 1 to 2, wherein the pair (33) of actuation members (24, 24') is suitable for being placed from the free configuration thereof to the actuation configuration thereof by a relative movement of the actuation members (24, 24') with respect to the base (132), and preferably wherein the first and second positions define a first degree of freedom, wherein the pair of actuation members is also movably mounted with respect to the base according to a third degree of freedom from the free configuration thereof to the actuation configuration thereof.

4. The robotised actuation module according to any one of claims 1 to 3, wherein la base (132) is a first base, the pair (33) of actuation members (24, 24') is a first pair of actuation members, the robotised module further comprising:
- a second base (132'),
- a second pair (33') of actuation members (24", 24"') each having an actuation surface (34", 34‴), the second pair (33') of actuation members (24", 24‴) being suitable for being placed alternately in an actuation configuration wherein the actuation surfaces (34", 34"') of the actuation members (24", 24‴) of the second pair (33') of actuation members (24", 24"') are engaged with the elongated flexible medical device to be actuated and arranged on either side thereof, and in a free configuration wherein the actuation surface (34", 34‴) of the actuation members (24", 24"') of the second pair (33') of actuation members (24", 24‴) is not engaged with the elongated flexible medical device,
the second pair (33') of actuation members (24", 24"') being movably mounted with respect to the second base (132') according to a degree of freedom between a first and a second positions,
- the control member (18, 11) being further suitable for controlling in a cyclically repeated manner a movement with respect to the base (132') of the actuation members (24", 24‴) of the second pair (33') of actuation members (24", 24‴) in the actuation configuration from the first to the second position, thus actuating the elongated flexible medical device with respect to the second base (132'), and a movement with respect to the second base (132') of the actuation members (24", 24‴) of the second pair (33') of actuation members (24", 24‴) in the free configuration from the second to the first position without actuating the flexible medical device with respect to the second base (32').

5. The robotised actuation module according to claim 4, wherein the first base (132) and the second base (132') are rigidly connected or common.

6. The robotised actuation module according to claim 4 or 5, wherein the control member (18, 11) is suitable for controlling the movements of the actuation members of the first pair and of the second pair in a synchronised manner.

7. The robotised actuation module according to claim 6, wherein the control member (18, 11) is suitable for placing the actuation members of the first pair and of the second pair simultaneously in the actuation configuration, and/or wherein the control member (18, 11) is suitable for placing the actuation members of the first pair and of the second pair simultaneously in the free configuration, and/or wherein the control member (18, 11) is suitable for placing simultaneously the actuation members of the first pair and of the second pair in one case in the actuation configuration and in the other in the free configuration.

8. The robotised module for actuating a movable element in the form of an elongated flexible medical device according to any one of claims 1 to 7, **characterised in that** it comprises a movement transmission chain comprising:
- a base block (640) of a member (24, 24') for actuating a movable element (15', 15"),
- three actuators (610, 620, 630) controlling the base block (640) of the actuation member (24, 24') respectively along three mutually distinct translation directions (Y, X, Z), via three respective interfaces (612, 622, 632) with the base block (640) of the actuation member (24, 24'),
and **in that** the intersection of the mean surface areas of the three interfaces (612, 622, 632) is located in the central region of the base block (640) of the actuation member (24, 24').

9. The robotised module for actuating a movable element in the form of an elongated flexible medical device according to claim 8, **characterised in that**:
- the three interfaces (612, 622, 632) are substantially planar,
- these three interfaces (612, 622, 632) are orthogonal with respect to one another,
- and these three interfaces (612, 622, 632) are interlocked in one another.

10. The robotised actuation module according to any one of the preceding claims, **characterised in that** it also comprises at least:
- an actuator (711, 741) controlling a pair of actuation members,
- an intermediate part transmitting the movement of the actuator (711, 741) to the pair of actuation members, so as to translate in opposite directions the two actuation members of the pair of actuation members, while keeping the distance between the two actuation members of the pair of actuation members substantially constant, so as to rotate an elongated flexible medical device about itself when the latter is arranged between the two actuation members of the pair of actuation members.

11. The robotised actuation module according to claim 10, **characterised in that**:
- the intermediate part is a rocker converting a translation of the actuator (711, 741) along a first direction (H) into two translations in opposite directions of the two respective actuation members along a second direction (V) orthogonal to the first direction (H).

12. The robotised actuation module according to claim 11, **characterised in that**:
- the rocker comprises a plate (700) which is connected to the actuator and which has two inclined oblong holes (701, 702) of opposing inclination in which at least two lugs (703, 704) respectively connected to the actuation members slide, the inclination of the oblong holes (701, 702) being closer to the first direction (H) than the second direction (V).

13. The robotised actuation module according to claim 11, **characterised in that**:
- the rocker comprises a plate (700) which is connected to the actuator and which has two inclined oblong holes (701, 702) of opposing inclination in which at least two rollers (707, 708, 709, 710) respectively connected to the actuation members slide, the inclination of the oblong holes (701, 702) being closer to the first direction (H) than the second direction (V).

14. The robotised actuation module according to claim 11, **characterised in that**:
- the rocker comprises a plate (716) which is connected to the actuator (711) and which has two inclined oblique rails (714, 715) of opposing inclination in which at least two slides (714', 715') respectively connected to the actuation members slide, the inclination of the rails (714, 715) being closer to the first direction (H) than the second direction (V).

15. The robotised actuation module according to claim 14, **characterised in that**:
- the two rails (714, 715) are in the same plane parallel to the plane formed by the first direction (H) and by the second direction (V).

16. The robotised actuation module according to claim 14, **characterised in that**:
- the two rails (723, 724) are in two distinct planes perpendicular to the plane formed by the first direction (H) and by the second direction (V).

17. The robotised actuation module according to claim 10, **characterised in that**:
- the rocker is pivoting about an axis (740) and comprises a plate (730) which is connected to the actuator and which has three inclined oblong holes (734, 735, 736) of the same inclination in which at least three lugs (737, 738, 739) or three rollers respectively connected to the actuator and to the actuation members slide, the inclination of the oblong holes (734, 735, 736) being closer to the first direction (H) than the second direction (V), two of the oblong holes (735, 736) being arranged symmetrically with respect to the pivoting axis (740) and receiving the lugs (738, 739) or the rollers respectively connected to the two actuation members, the third oblong hole (734) being arranged further from the pivoting axis (740) than the two oblong holes (735, 736) connected to the actuation members and receiving the lug (737) or the roller connected to the actuator.

18. The robotised actuation module according to claim 11, **characterised in that**:
- the rocker comprises a plate (751) which is connected to the actuator (741) and which has two connecting rod (743, 744) and L-shaped crankshaft (748, 749) systems, the two L-shaped crankshafts (748, 749) being oriented in opposite directions, the small portion of the L of the crankshafts (748, 749) being substantially along a first direction (H), the large portion of the L of the crankshafts (748, 749) being substantially along the second direction (V).

19. The robotised actuation module according to claim 11, **characterised in that**:
- the rocker comprises a plate (752) which is connected on one side to the actuator and which is connected on the other side to a first end (762) of a connecting rod (753) the second end (763) whereof is connected to a first end (763) of a first rod (756) sliding at the centre (760) thereof in a first oblong hole (758) located at a first end of a bar (754) pivoting at the middle (755) thereof and the second end whereof has a second oblong hole (759) in which the middle (761) of a second rod (757) slides, the oblong holes (758, 759) being parallel to the bar (754), the second ends (764, 765) of the two rods (756, 757) being respectively connected to the actuation members.

20. The robotised actuation module according to claim 11, **characterised in that**:
- the rocker comprises a plate (770) which is connected to the actuator and which has a first rack (771) along the first direction (H), two second racks (772, 773) which are respectively connected to the actuation members and which are along the second direction (V) and the toothed portions whereof face one another, two gear systems located between the first rack (771) and the respective two second racks (772, 773), each of the gear systems comprising a large gear (776, 777) engaging with the first rack (771) and a small gear (774, 775) engaging with one of the second racks (772, 773).
